# EUROPEAN PATENT SPECIFICATION

(11) **EP 1 458 388 B1**
(45) Date of publication and mention of the grant of the patent: **01.04.2009**
(21) Application number: 02793665.7
(22) Date of filing: 18.12.2002
(51) Int. Cl.: A61K 31/465, A61P 25/34

(54) **A LIQUID PHARMACEUTICAL FORMULATION COMPRISING NICOTINE FOR THE ADMINISTRATION TO THE ORAL CAVITY**
FLÜSSIGE PHARMAZEUTISCHE ZUSAMMESETZUNG MIT NIKOTIN ZUR VERABREICHUNG IN DIE MUNDHÖHLE
PREPARATION PHARMACEUTIQUE LIQUIDE RENFERMANT DE LA NICOTINE, DESTINEE A ETRE ADMINISTREE PAR VOIE ORALE

(30) Priority: 27.12.2001 SE 0104388
(43) Date of publication of application: 22.09.2004
(60) Divisional application: 08021421.6
(73) Proprietor: McNeil AB, 25109 Helsingborg (SE)
(72) Inventor: LINDELL, Katarina, S-241 93 Eslöv (SE); BOSSON, Bengt, S-256 55 Helsingborg (SE); BERGENGREN, Gunnar, S-267 32 Bjuv (SE); SCHLÜTER, Anette, S-257 32 Rydebäck (SE)
(74) Representative: Hedenström, John Allan
(86) International application number: PCT/SE2002/002358
(87) International publication number: WO 2003/055486

(56) References cited:
- EP-B- 0 324 794
- FR-A- 2 792 200
- GB-A- 2 255 892
- US-A- 5 721 257
- US-A- 5 955 098
- MOLANDER L. ET AL.: 'Pharmacokinetic investigations of a nicotine sublingual tablet' EUR. J. CLIN. PHARMACOL. vol. 56, 2001, pages 813 - 819, XP002959079
- GARRETT BRIDGETTE E. ET AL.: 'Tobacco addiction and pharmacological interventions' EXPERT OPIN. PHARMACOTHER. vol. 2, no. 10, 2001, pages 1545 - 1555, XP002959080
- A. Axelsson and Bo Brentmark, THE ANTI-SMOKING EFFECT OF CHEWING GUM WITH NICOTINE OF HIGH AND LOW BIOAVAILABILITY, DHEW, Proceedings from the 3rd World Conference on Smoking and Health, New York 1975, Publication No. (NIH) 771413, pages 549-559
- NICORETTE, The Nicotine Gum Monograph, Adis International, Chester, 1992, pages 1 to 19

## Description

### Technical Field

This invention relates to a liquid pharmaceutical formulation for delivering nicotine to a subject. This invention also relates to a method and a system for delivering nicotine as well as manufacturing and use of said liquid pharmaceutical formulation.

### Background of the Invention

### Tobacco dependence and reduction thereof

In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief active ingredient in the tobacco used in cigarettes, cigars, snuff and the like. Nicotine is also an addictive drug, though, and smokers characteristically display a strong tendency to relapse after having successfully stopped smoking for a time. Nicotine is the world's second most used drug, after caffeine from coffee and tea.

The main problem with tobacco smoking is its enormous implications on health. Today it is estimated that smoking-related diseases cause some 3 - 4 million deaths per year. In the US Surgeon General's 1988 report on The Health Consequences of Smoking, it was estimated that in the US alone about 300.000 deaths are caused each year by diseases related to cigarette smoking. In fact, excessive smoking is now recognized as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug.

The most advantageous thing a heavy smoker can do is to reduce or preferably even stop smoking completely. Experience shows, however, that most smokers find this extremely difficult since, mostly, tobacco smoking result in a dependence disorder or craving. The WHO has in its International Classification of Disorders a diagnosis called Tobacco Dependence. Others, like the American Psychiatric Association call the addiction Nicotine Dependence. It is generally accepted that these difficulties to stop smoking result from the fact that those heavy smokers are dependent on nicotine. The most important risk factors are, however, substances that are formed during the combustion of tobacco, such as carcinogenic tar products, carbon monoxide, aldehydes, and hydrocyanic acid.

### Effects of nicotine

The administration of nicotine can give satisfaction and the usual method is by smoking, either by smoking e g a cigarette, a cigar or a pipe, or by snuffing or chewing tobacco. However, smoking has health hazards and it is therefore desirable to formulate an alternative manner of administering nicotine in a pleasurable manner that can be used to facilitate withdrawal from smoking and/or used as a replacement for smoking.

Upon smoking of a cigarette, nicotine is quickly absorbed into the smoker's blood and reaches the brain within around ten seconds after inhalation. The quick uptake of nicotine gives the consumer a rapid satisfaction, or kick. The satisfaction, then, lasts during the time of smoking the cigarette and for a period of time thereafter. The poisonous, toxic, carcinogenic, and addictive nature of smoking has provided efforts for methods, compositions and devices, which help in breaking the habit of smoking.

Nicotine is an addictive poisonous alkaloid C₅H₄NC₄H₇NCH₃, derived from the tobacco plant. Nicotine is also used as an insecticide. Approximately forty milligrams of nicotine may kill an adult (Merck Index).

### Nicotine replacement products

One way to reduce smoking is to provide nicotine in a form or manner other than by smoking and some products have been developed to fulfill this need. Nicotine containing formulations are currently the dominating treatments for tobacco dependence.

The success in achieving reduction in the incidence of smoking has been relatively poor using presently known products. State of the art involves both behavioral approaches and pharmacological approaches. More than 80% of the tobacco smokers who initially quit smoking after using some behavioral or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market, such as nicotine chewing gums according to US 3,845,217. Several methods and means have been described for diminishing the desire of a subject to use tobacco, which comprises the step of administering to the subject nicotine or a derivative thereof as described in e g US 5,939,100 (nicotine containing microspheres) and US 4,967,773 (nicotine containing lozenge).

The effects of pH on the absorption of nicotine is discussed e g in Eur J Clin Pharmacol, Vol. 56, 2001, pages 813 - 818, L. Molander et al, "Pharmacokinetic investigation of a nicotine sublingual tablet". The effects of pH on a liquid nicotine formulation for administration to the oral cavity are though not disclosed.

The use of skin patches for transdermal administration of nicotine has been reported (Rose, in Pharmacological Treatment of Tobacco Dependence, (1986) pp. 158 - 166, Harvard Univ. Press). Nicotine-containing skin patches that are in wide use today can cause local irritation and the absorption of nicotine is slow and affected by cutaneous blood flow.

Nicotine-containing nose drops have been reported (Russell et al., British Medical Journal, Vol. 286, p. 683 (1983); Jarvis et al., British Journal of Addiction, Vol. 82, p. 983 (1987)). Nose drops, however, are difficult to administer and are not convenient for use at work or in other public situations. Ways of administrating nicotine by way of delivery directly into the nasal cavity by spraying is known from US 4,579,858, DE 32 41437 and WO/93 127 64. There may, though, be local nasal irritation with use of nasal nicotine formulations. The difficulty in administration also results in unpredictability of the dose of nicotine administered.

GB 2 255 892 discloses a tabletted smoking substitute formulation comprising a pharmaceutically acceptable salt of nicotine.

Also, inhaling devices resembling a cigarette are known for uptake of nicotine vapors as suggested in US 5,167,242. An aerosol for deposing nicotine in the lungs is disclosed in DE 32 41 437.

Mouth sprays comprising nicotine are known in the art, e g according to US 6,024,097 wherein is disclosed a method of assisting a smoker in giving up the smoking habit whereby is used a plurality of aerosol dispensers comprising progressively lesser concentrations of nicotine. The aerosol is intended to be administered into the mouth. The liquid in the dispensers essentially consists of nicotine and alcohol.

A similar mouth spray is disclosed in US 5,810,018, whereby in addition the aerosol comprises progressively greater concentrations of at least one selected stimulant.

WO 98/24420 discloses an aerosol device with an active and a propellant. The device may be used for e g sublingual administration. Nicotine is mentioned as an active in a long "laundry list" of drugs. There are though no examples on nicotine formulations.

US 5,721,257 discloses a method for treating a condition responsive to nicotine therapy comprising a first treatment with transdermally administered nicotine and a second treatment with transmucosally administered nicotine. It is stated that the transmucousal administration may be accomplished via an aerosol to the nasal membranes. No administration to the oral cavity is disclosed.

WO 97/38663 discloses a buccal aerosol spray using a non-polar solvent. Nicotine is mentioned as one useful active in this spray.

US 5,955,098 likewise discloses a buccal non-polar spray wherein nicotine may be an active.

None of the known mouth sprays comprise any buffering and/or pH regulating means.

### Prior art and problems thereof

The captioned means and methods do not satisfy the craving that certain users of tobacco experience. Specifically these means and methods generally do not provide for a sufficiently rapid uptake of nicotine without adverse effects.

This means that none of the hitherto known means and methods satisfactorily fulfills the following well-known NRT teaching by Russel et al:
I: A fast delivery or "boost" of nicotine, sufficiently rapid to give positive subjective nicotine effects in contrast with current nicotine gums and patches, will lead to faster craving relief, and
II: faster craving relief will give better craving control, and
III: better craving control should result in higher overall quit rates.

For the captioned see Russel, M.A.H., Stapleton, J.A. and Feyerabend C. Nicotine boost per cigarette as the controlling factor of intake regulation of smokers; In: Clark et al. (Eds.) Effects of Nicotine on Biological Systems II, Advances in Pharmacological Sciences, Birkhäuser Verlag, Basel, (1995) 233-238.

In light of the aforementioned problems there is a strong need and interest to develop means and methods for the administration of nicotine to provide a fast satisfaction to a person craving for nicotine or to provide a sense of smoking satisfaction without smoking, whereby also may be avoided problems associated with the prior art means and methods. The present invention addresses said need and interest.

### Summary of the Invention

In view of the foregoing disadvantages known in the art when trying to deliver nicotine to a subject so as to obtain a rapid transmucosal uptake of nicotine in the oral cavity of the subject the present invention provides a new and improved product, systems and methods for obtaining a rapid transmucosal uptake of nicotine in the oral cavity of the subject.

Objects of the present invention are to provide an efficient and effective product, as well as methods and systems for a rapid uptake of nicotine in a subject to avoid the disadvantages of previously known products and methods. The present invention also satisfactorily satisfies the above teaching of Russel et al.

Thus, the present invention provides a liquid pharmaceutical formulation containing nicotine for administration to the oral cavity of a subject as well as a method for manufacturing said liquid pharmaceutical formulation.

Furthermore, the present invention provides a system for delivering nicotine to a subject, comprising said liquid pharmaceutical formulation and at least one other means for obtaining reduction of the urge to smoke or use of tobacco as well as a system for obtaining reduction of the urge to smoke or otherwise use of tobacco and/or for providing a sense of smoking satisfaction without smoking, comprising a liquid pharmaceutical formulation as per above and at least one other method for obtaining reduction of the urge to smoke or otherwise use tobacco. Said system may be a system wherein the at least one other method is selected from the group consisting of administration through chewing gums, nasal sprays, transdermal patches, inhaling devices, lozenges, tablets and parenteral methods, subcutaneous methods, intravenous methods, rectal methods, vaginal methods and transmucousal methods; or other use of tobacco.

The present invention provides for a flexible, convenient and discrete use in comparison with other means for transmucosal delivery of nicotine, e g chewing gums, lozenges and tablets. No chewing or sucking is necessary. Further and in contrast to other transmucosal dosage forms the present liquid pharmaceutical formulation provides nicotine in a form being directly buccally absorbable by a subject. Known formulations for nasal delivery of nicotine are inconvenient - side effects include running nose, nasal irritation and irritation of the eyes. The nicotine in chewing gums, lozenges and tablets need pass a transformation phase, involving e g mastication, disintegration, melting and/or dissolution, prior to being present in a directly absorbable form. A nicotine patch provides for a discrete administration, but does not provide for a fast uptake of nicotine.

A product according to the invention is as per Claim 1.

Use of said product will according to the invention rapidly deliver nicotine to a subject and will also provide for obtaining a quick and/or sustained and/or complete reduction of the urge to smoke or use tobacco and/or for providing a sense of smoking satisfaction without smoking resembling the sense of smoking satisfaction obtained after regular smoking or use of tobacco.

### Legend of Figures

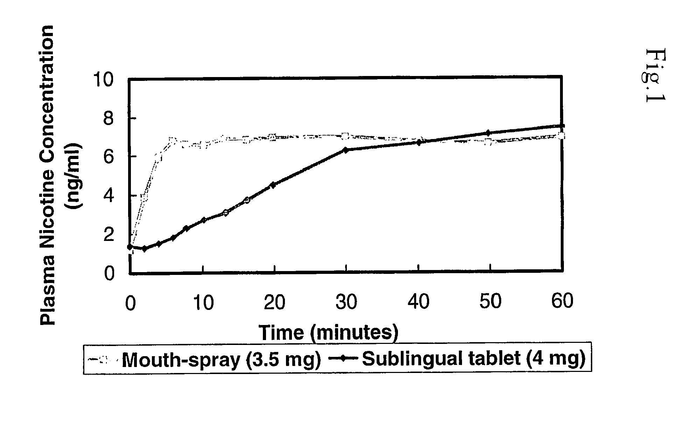

Figure 1 is a diagram showing venous blood plasma level concentrations of nicotine after two different ways of administering nicotine. For both ways of administration one unit dose was administered at time zero. No further doses were administered. 50 persons, all being nicotine users, took part in this test. "Spray" represents 200 µl of a liquid pharmaceutical formulation according to below Example 4 being sprayed under the tongue. This unit dose comprised 3.5 mg nicotine measured as free base. "Microtab" represents one tablet of Nicorette^{®} Microtab, comprising 4 mg nicotine measured as free base. Nicorette^{®} Microtab is pharmacologically equivalent to Nicorette^{®} Gum. "Spray" comprises a buffer. "Microtab" comprises no buffer. With "Spray" the liquid pharmaceutical formulation was held in the mouth for one minute before swallowing. With "Microtab" the tablet was kept under the tongue until dissolved. Each symbol on the respective graph represents one measurement of nicotine in venous blood plasma.

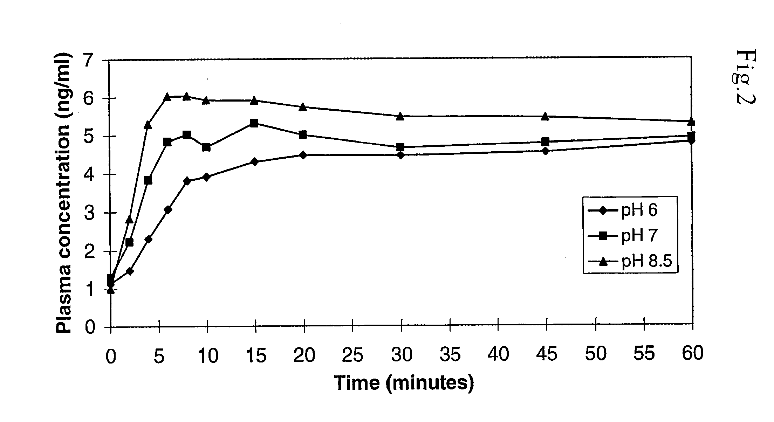

Figure 2 shows mean plasma concentrations after sublingual administration of three liquid pharmaceutical formulations with pH 6, 7 and 8.5 respectively. For each formulation 200 µl was sprayed under the tongue at time zero. For all said three formulations the concentration of nicotine was 10 mg/ml, i e each 200 µl spray dose as above contained 2 mg nicotine calculated as free base. The formulation with pH 8.5 was a formulation according to below Example 1. The formulations with pH 7 and pH 6 were formulations according to below Example 2 and Example 3 respectively.

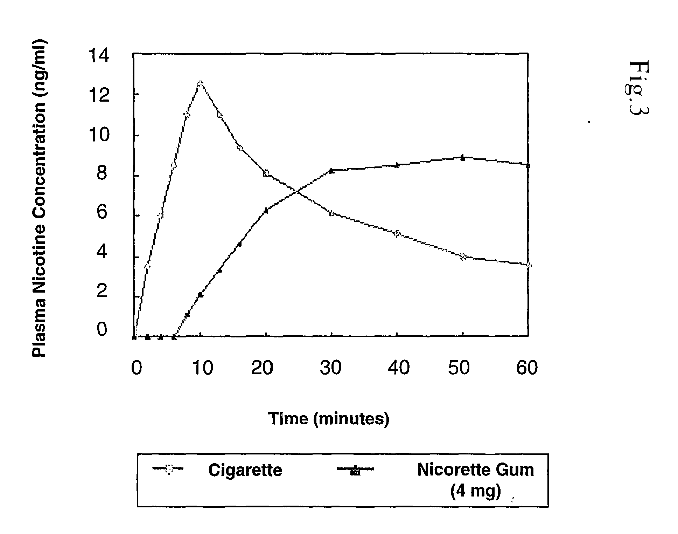

Figure 3 compares the venous blood nicotine plasma profile vs. time of a single dose of the current Nicorette^{®} Gum, extra strength (4 mg), with the corresponding plasma profile when smoking a "light" (low-nicotine) cigarette. One objective with the present invention is to obtain a buccal nicotine formulation providing for a pK profile being closer to the pK profile for a cigarette than is provided using presently known buccal nicotine formulations.

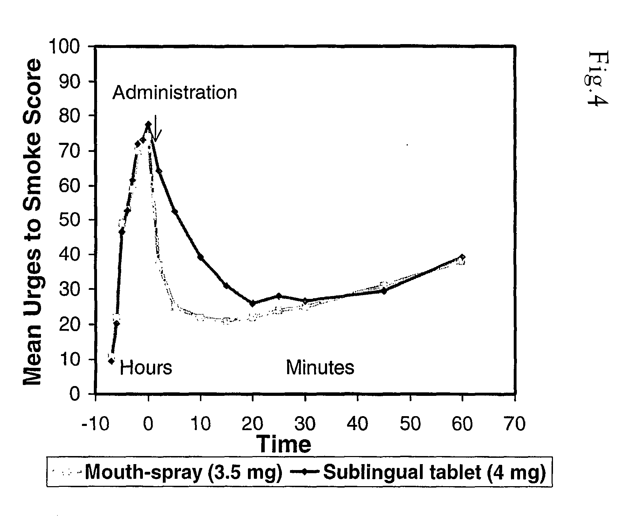

Figure 4 shows the mean score values from 52 smoking volunteers in a randomized open study of the "urge to smoke" (craving), as estimated and recorded on a visual analogue scale (VAS) as a function of time when the same formulations as in Fig. 1 were used. The craving scores were recorded directly after smoking one cigarette and during the abstinence of 7 hours before the administration of the nicotine products. The scores were then recorded more frequently during 1 hour after the administration. The heart rate was also monitored in this study. This figure clearly shows that the present invention provides for a much faster reduction of the urge to smoke score than do present buccal nicotine formulations. For example, about 2 minutes after administration of a formulation according to the present invention the craving score is reduced by 50 %. With Nicorette^{®} Microtab a 50 % decrease in craving score is obtained only more than 10 minutes after administration.

### Detailed Description of the Invention

### Definitions

The terms "tobacco", "tobacco containing material" and similar are herein intended to mean such material for any type of use of tobacco including smoking, snuffing or chewing whereby is used inter alia a cigarette, a cigar, pipe tobacco, snuff and chewing tobacco.

The term "fast reduction of the urge to smoke or use tobacco " is herein intended to mean an initial priming of the subject so as to achieve a reduction of the urge to smoke or use tobacco.

The term "transient" is intended to pertain to a non-permanent change of a biological and/or physiological state, upon which after a certain period of time said state will return to its value or behavior prior to said change.

The term "buccal" and "buccally" are herein intended to pertain to all of or any part of the soft tissue lining of the oral cavity.

The term "liquid of the oral cavity" is herein intended to mean saliva and/or saliva mixed with a quantity of the liquid pharmaceutical formulation.

The term "incidence of administration" is herein intended to mean administration of one or more single doses of the liquid pharmaceutical formulation within the same time frame, said time frame being dependent on the needs of the subject receiving the administration, said time frame extending from a few seconds to around ten minutes.

### The buffering agent and the pH regulating means

Absorption of nicotine from the oral cavity to the systemic circulation is dependent on the pH of the saliva and the pKa of nicotine, which is about 7.8. Assuming a pH of the saliva of 6.8 only about 10% of the nicotine in saliva will be in the free base form. Thus, in order to promote absorption of nicotine in a free base form, which is the form predominantly absorbed through the mucosa, the pH of the saliva must be increased. At a pH of 8.8 about 90% of the nicotine in saliva will then be in the free base form.

Hence and according to the invention, the liquid pharmaceutical formulation is alkalized by buffering and/or pH regulation. This may be achieved by including physiologically acceptable buffering substances or agents, or by other means. With other means it is intended to include buffering by any component in the product, which may not normally act as a buffering agent, such as a self-buffering additive and/or pH regulating forms of nicotine.

By buffering and/or pH regulation thereby increasing the pH of the saliva the uptake of nicotine is changed, e g increased compared to the nicotine uptake when the saliva is not alkalized by buffering and/or pH regulation. Also, since the transmucosal uptake of nicotine in the oral cavity according to the invention is faster than for nicotine not being buffered and/or pH regulated according to the invention, less nicotine will be swallowed and reach the gastrointestinal (GI) tract. The nicotine that reaches the GI tract will be subjected to first pass metabolism, which reduces the total amount of intact nicotine absorbed additionally reducing the rate of nicotine absorption. This means that the absorption kinetics of nicotine that is not co-administered with a buffer according to the invention will generally be slower and the bioavailability will generally be lower than when administered together with a buffer.

For buffering may be used one or more buffering agents selected from the group consisting of carbonates including bicarbonate or sesquicarbonate, glycinate, phosphate, glycerophosphate or citrate of an alkali metal, such as potassium or sodium, or ammonium, and mixtures thereof.

Further embodiments may use trisodium or tripotassium citrate, and mixtures thereof.

Still further embodiments may comprise different phosphate systems, such as trisodium phosphate, disodium hydrogen phosphate; and tripotassium phosphate, dipotassium hydrogen phosphate, and calcium hydroxide, sodium glycinate; and mixtures thereof.

Alkali metal carbonates, glycinates and phosphates are preferred buffering agents.

The pH regulation may also be obtained by using pH-regulating forms of nicotine, e g nicotine free base.

The amount of the buffering agent or agents in the liquid pharmaceutical formulation is preferably sufficient in the specific embodiments to raise the pH of the saliva to above 7, as specified above and, to maintain the pH of the saliva in the oral cavity above 7, e g pH 7 - 11. Otherwise expressed the liquid pharmaceutical formulation should be alkalized by buffering and/or pH regulation in such a way that upon administration to a subject the pH of the liquid of the oral cavity of the subject is transiently increased by about 0.3 - 4 pH units, preferably by about 0.5 - 2.5 pH units. The amount of buffering agent(s) required to achieve such an increase in pH is readily calculated by a person skilled in the art.

### The active ingredient

According to the invention, the liquid pharmaceutical formulation product comprises nicotine to provide a fast transmucosal uptake of the nicotine in the oral cavity of a subject so as to obtain a reduction of the urge to smoke and/or use tobacco, and/or a rapid "nicotine kick" and/or a "nicotine head rush". Thereby may also be achieved a systemic maintenance level of nicotine.

The nicotine should be in a saliva soluble form to facilitate the subsequent uptake of the nicotine from the saliva in the oral cavity into the systemic circulation of the subject.

According to the invention, the uptake of the nicotine through any tissue or mucosa in the oral cavity is improved in relation to the uptake obtained by a liquid nicotine-containing pharmaceutical formulation devoid of alkalizing buffering agents or devoid of alkalizing pH-regulating means.

The nicotine may act as a stimulant to e g obtain a rapid reduction of the urge to smoke or to use tobacco.

With nicotine it is intended to include nicotine, 3-(1-methyl-2-pyrrolidinyl)-pyridine, with its base form, including synthetic nicotine as well as nicotine extracts from tobacco plants, or parts thereof, such as the genus Nicotiana alone or in combination.

### Amount of the nicotine in the liquid pharmaceutical formulation

The nicotine is according to the invention formulated to provide the subject with a dose to achieve an effect. The effect may be to provide a sense of smoking satisfaction without smoking. Another effect of the administered nicotine in any form may be a reduction of the urge to smoke or use tobacco.

The effect may also be a combination of a reduction of said urge and providing a sense of smoking satisfaction without smoking. The amount of the nicotine should be sufficient to provide such an effect in a subject. This amount may, of course, vary from person to person.

According to the invention, embodiments of the liquid pharmaceutical formulation comprise nicotine in such concentrations that the amount of nicotine delivered at each incidence of administration is about 0.05 -10 mg calculated as the free base form of nicotine, preferably about 0.25 - 6 mg and most preferably about 0.5 - 4 mg.

### Release and uptake of nicotine

Presently existing pharmaceutical administration forms for oral administration of nicotine normally provide a slow release and a slow uptake of the nicotine compared to smoking. The slow uptake of the nicotine provides a tₘₐₓ, i e the time-point where the nicotine has its maximum level measured in the plasma of venous blood after a single dose at about 30 - 45 minutes after administration.

The time point for reaching a sense of satisfaction or reduction of urge to smoke or use tobacco after administration is individual, but may in existing pharmaceutical forms for administering nicotine generally be reached after approximately 30 minutes when regarded as coinciding with tₘₐₓ, According to the present invention, such a sense of satisfaction may be reached after a shorter period of time due to a rapid transmucosal uptake in the oral cavity due to the buffering and/or pH regulation and due to the absence of rate-limiting steps, such as tablet or lozenge melting, tablet or lozenge disintegration and dissolution and chewing gum mastication, followed by drug dissolution.

### The liquid phase

The liquid phase of the present liquid pharmaceutical formulation may comprise water. The liquid phase may also comprise an alcohol, such as ethanol, glycerol, propylene glycol and polyethylene glycol, or mixtures thereof. It may also comprise one or more lipids. Further it may comprise mixtures of the above ingredients.

### Other additives to the liquid pharmaceutical formulation

Other additives may be added optionally to the liquid pharmaceutical formulation.

Optional additives comprise one or more stabilizing additives, such as those selected from the group consisting of antioxidants including vitamin E, i e tocopheroles, vitamin C, i e ascorbic acid and its salts, sodium pyrosulfite, butylhydroxytoluene, butylated hydroxyanisole; and preservatives including parabenes, benzalkonium chloride, chlorbutanol, benzyl alcohol, beta-phenylethylal alcohol, cetylpyridinium chloride; and chelating agents, such as EDTA; and galates, such as propyl galate.

Further optional additives comprise one or more additives selected from the group consisting of:
- enhancers, such as azone;
- vitamins, such as vitamins C and E;
- minerals, such as fluorides, especially sodium fluoride, sodium monofluoro phosphate and stannous fluoride;
- anti-odours, such as zinc and cyclodextrins;
- sweeteners including one or more synthetic sweetening agents and/or natural sugars, such as those selected from the groups consisting of artificial sweeteners e g saccharin and its sodium and calcium salts, aspartame, acesulfame and its potassium salt, thaumatin and glycyrrhizin;
- polyhydric alcohols such as sorbitol, xylitol, mannitol and glycerol;
- monosaccharides including glucose (also called dextrose), fructose (also called laevulose) and galactose;
- disaccharides including saccharose (also called sucrose), lactose (also called milk sugar) and maltose (also called malt sugar);
- mixtures of sugars including liquid glucose syrup e g starch hydrolysates containing a mixture of chiefly dextrose, maltose, dextrins and water, invert sugar syrup e g sucrose inverted by invertase containing a mixture of dextrose, laevulose and water, high sugar content syrups such as treacle, honey and malt extract; and mixtures thereof;
- flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of peppermint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary;
- natural flavors and aroma agents including either diluted solutions of essential oils or concentrates of flavor components with natural origin from e g fruits, berries, nuts, spices, mints, tobacco, cocoa, coffee, tea, vanilla, liquorice, caramel, toffee, honey, wine, liquors and brews;
- synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews;
- and mixtures thereof.

### Surface active agents

One or more of the compounds of the liquid pharmaceutical formulation may be solubilized in one or more surface active agents and/or emulsifiers, such as nonionic, cationic, anionic or zwitterionic surfactants, including amphiphilic block copolymers, or mixtures thereof.

Specifically one or more of the compounds of the liquid pharmaceutical formulation may be solubilized in one or more surface-active agents selected from nonionic surface-active agents including poloxamers, e g:
- poly (oxypropylene)-poly (oxyethylene) block copolymers, polyoxyethylene alkyl ethers, polyoxyethylene castor oil derivatives, polyoxyethylene sorbitan fatty acid esters, mono- and diglycerides and esters thereof, polyoxyethylene stearates, polyglycerolesters of fatty acids (including polyglycerolpolyricinoleic acid (PGPR)), and sorbitan fatty acid esters,
- cationic surface-active agents including secondary, quaternary and tertianary ammonium compounds and cationic phospholipids,
- anionic surface-active agents including fatty acid salts, lactylates, especially sodium and/or calcium stearoyllactylate, alkyl sulphates, alkyl sulphonates, latanol, and anionic phospholipids, such as phosphatidylserine,
- zwitterionic surface-active agents including zwitterionic phospholipids, such as phosphatidylcholine and phosphatidylethanolamine,
- or mixtures thereof,
- preferably surface-active agents or mixtures thereof being nonionic.

### Method for delivering nicotine in any form to a subject

According to the invention, a method for delivering nicotine to a subject comprises the steps of:
a) administering to a subject a liquid pharmaceutical formulation product containing nicotine according to the invention into the oral cavity of the subject, and
b) allowing the nicotine in the liquid pharmaceutical formulation to be mixed with the saliva in the oral cavity and absorbed into the blood plasma of the subject essentially by buccal uptake.

One embodiment results in a tₘₐₓ of nicotine in venous blood of the subject after about 3 - 30 minutes.

One further embodiment results in a tₘₐₓ of nicotine in venous blood of the subject after about 3 - 20 minutes.

In still one further embodiment, said nicotine is absorbed resulting in a tₘₐₓ of nicotine in venous blood of the subject after about 3 -15 minutes.

### Method for obtaining reduction of the urge to smoke or use tobacco

A method for obtaining reduction of the urge to smoke or use tobacco-containing material and/or for providing a sense of smoking satisfaction without smoking according to the invention comprises the steps of:
a) replacing at least partly the tobacco containing material with a liquid pharmaceutical formulation according to any of claims 1 - 22,
b) administering to a subject a liquid pharmaceutical formulation containing nicotine according to any of claims 1 - 22 into the oral cavity of the subject, and
c) allowing the nicotine in the liquid pharmaceutical formulation to be absorbed by the subject essentially by buccal uptake.

The administration to the oral cavity takes place by spraying, dropping or pipetting, preferably by spraying, most preferably by spraying under the tongue. The administration is intended for the oral cavity, not for e g the lungs or the upper respiratory tract.

In one embodiment said nicotine results in a tₘₐₓ of nicotine in venous blood of the subject after about 3 - 30 minutes.

In one further embodiment said nicotine results in a tₘₐₓ of nicotine in venous blood of the subject after about 3 - 20 minutes.

In still one further embodiment said nicotine results in a tₘₐₓ of nicotine in venous blood of the subject after about 3 - 15 minutes.

Even further embodiments of the method for delivering nicotine to a subject may comprise the steps of combining at least one other method for obtaining reduction of the urge to smoke or use of tobacco.

The liquid pharmaceutical formulation may be used for obtaining a quick and/or sustained and/or complete reduction of the urge to smoke or use tobacco and/or for providing a sense of smoking satisfaction without smoking as further discussed below.

The fast relief provides the subject with a sense of rapid smoking satisfaction without smoking.

One embodiment reduces the urge to smoke or use of tobacco by reaching a tₘₐₓ of nicotine in venous blood of the subject after about 3 - 30 minutes by the use of a liquid pharmaceutical formulation according to the invention.

### Cessation of the urge to smoke or use tobacco

For some of the users, it may be a goal to terminate the usage of nicotine completely, due to several reasons e g health, economical, social or behavioral. This may be achieved by further decreasing the delivered amount of nicotine gradually over time. In specific embodiments of the invention, the craving relief may further comprise the steps of decreasing the amount of nicotine in the liquid pharmaceutical formulation gradually over time, and/or the steps of reducing the incidence of administration of the liquid pharmaceutical formulation gradually over time, and/or the steps of reducing the dosage size of the liquid pharmaceutical formulation gradually over time, so as to achieve a relief of tobacco craving and/or to achieve a sense of smoking satisfaction. This method results in a weaning process gradually over time.

Different types of smokers reach the sense of reduced craving at different plasma levels of nicotine. This may, of course, affect the individual types of programs for administering a liquid pharmaceutical formulation according to the invention. Different types of smokers include e g peak seekers or smokers that crave for a plasma level of nicotine constantly being above the level below which withdrawal symptoms occur.

One strategy may be to lower the frequency of administering the liquid pharmaceutical formulation. Other embodiments include varying the dose of the nicotine in said liquid pharmaceutical formulation as well as the combination of these two embodiments.

### Systems for delivering nicotine and for obtaining craving relief

According to the invention there is a system for delivering nicotine to a subject. Such a system comprises a liquid pharmaceutical formulation according to the invention and at least one other means for obtaining reduction of the urge to smoke.

Another system according to the invention may be a system for obtaining reduction of the urge to smoke or use tobacco and/or for providing a sense of smoking satisfaction without smoking. Such a system comprises a liquid pharmaceutical formulation according to the invention and at least one other method for obtaining reduction of the urge to smoke or use tobacco. Other methods may be a concomitant or concurrent method selected from the group consisting of administration through chewing gums, nasal sprays, transdermal patches, inhaling devices, lozenges, tablets and parenteral methods, subcutaneous methods, intravenous methods, rectal methods, vaginal methods and transmucosal methods; or use of tobacco.

In a specific embodiment, the at least one other method comprises administration of nicotine.

### Use of the liquid pharmaceutical formulation

The use of the liquid pharmaceutical formulation according to the invention is for obtaining a fast and/or complete reduction of the urge to smoke and use tobacco or for providing a sense of smoking without smoking as described above.

The dose of the nicotine is chosen to give the subject an individual sensory perception and satisfaction with an effect of the nicotine in any form. The use of the liquid pharmaceutical formulation may also be a sole use according to the invention or a combination with other means or methods known in the field of drug abuse. Specifically, the present invention may be used in combination with other means as described above in the methods in the paragraphs above.

The use may give a quick reduction of the urge to smoke or use tobacco whereby is reached a tₘₐₓ of nicotine in venous blood after about 3 - 20 minutes.

In a specific embodiment, the use of the liquid pharmaceutical formulation according to the invention will reduce the urge to smoke or use tobacco by reaching a tₘₐₓ of nicotine in venous blood of the subject after about 3-15 minutes.

According to the invention, a use of a liquid pharmaceutical formulation according to the invention is for delivering nicotine to a subject.

In one embodiment, the delivering of nicotine results in a tₘₐₓ of nicotine in venous blood of the subject after about 3-30 minutes.

In another embodiment, the delivering of nicotine results in a tₘₐₓ of nicotine in venous blood of the subject after about 3-20 minutes.

In still another embodiment, the delivering of nicotine results in a tₘₐₓ of nicotine in venous blood of the subject after about 3-15 minutes.

As readily shown and concluded from the figures, e g Figure 4, the shorter the tₘₐₓ the faster the relief of the craving, i e of the urge to smoke.

### Examples on embodiments and manufacturing of the liquid pharmaceutical formulation

The below examples are non-limiting and for illustrating the present invention. Alternatives and variations of the below examples within the scope of the present invention as per the below claims may be carried out by a person skilled in the art. Ingredients as per the below examples may be exchanged for equivalent ingredients, preferably as per above. The formulations according to Examples 2 and 3 were made for comparative purposes as seen from Figure 2.

### Example 1

Manufacturing of a 1000 ml formulation with 10 mg nicotine/ml and around pH 8.5.

### Mixture 1

To a beaker containing 800 ml water of 90°C was added 0.7 g methyl para-hydroxybenzoate, acting as preservative, and 0.3 g propyl para-hydroxybenzoate, acting as preservative. The additives were dissolved during stirring for about 10 minutes. Then was added 10.45 g sodium dihydrogen phosphate, acting as buffering agent, and 0.5 g EDTA, acting as chelating agent, to the solution, which was stirred for about 5 minutes. Then the solution was cooled to 30°C during stirring.

### Mixture 2

To a beaker containing 15.9 g ethanol of room temperature, acting as solvent, was added 0.045 g peppermint oil, acting as flavoring agent. The liquid was mixed for 2 minutes.

### Final mixture

Mixture 2 was added during stirring to a beaker containing 150 ml water. Gently 10 g nicotine (base) was added to the beaker. Then Mixture 1 was added to the beaker and stirred for 5 minutes. The pH of the Final mixture was checked and adjusted to about pH 8.5 with sodium hydroxide (20%) and to volume with water.

### Example 2

Manufacturing of a 1000 ml formulation with 10 mg nicotine/ml and around pH 7.0. This formulation is for comparative purpose.

This Example 2 differs from Example 1 only for pH. The formulation according to Example 2 contains a non-alkalizing buffering agent. This formulation was for use as a comparison in Figure 2.

### Mixture 1

To a beaker containing 800 ml water of 90°C was added 0.7 g methyl para-hydroxybenzoate, acting as preservative, and 0.3 g propyl para-hydroxybenzoate, acting as preservative. The additives were dissolved during stirring for about 10 minutes. Then was added 10.45 g sodium dihydrogen phosphate, acting as buffering agent, and 0.5 g EDTA, acting as chelating agent, to the solution, which was stirred for about 5 minutes. Then the solution was cooled to 30°C during stirring.

### Mixture 2

To a beaker containing 15.9 g ethanol of room temperature, acting as solvent, was added 0.045 g peppermint oil, acting as flavoring agent. The liquid was mixed for 2 minutes.

### Final mixture

Mixture 2 was added during stirring to a beaker containing 150 ml water. Gently 10 g nicotine (base) was added to the beaker. Then Mixture 1 was added to the beaker and stirred for 5 minutes. The pH of the Final mixture was checked and adjusted to about pH 7.0 with hydrochloric acid and to volume with water.

### Example 3

Manufacturing of a 1000 ml formulation with 10 mg nicotine/ml and around pH 6.0. This formulation is for comparative purpose.

This Example 3 differs from Example 1 only for pH. The formulation according to Example 3 contains a non-alkalizing buffering agent. This formulation was for use as a comparison in Figure 2.

### Mixture 1

To a beaker containing 800 ml water of 90°C was added 0.7 g methyl para-hydroxybenzoate, acting as preservative, and 0.3 g propyl para-hydroxybenzoate, acting as preservative. The additives were dissolved during stirring for about 10 minutes. Then was added 10.45 g sodium dihydrogen phosphate, acting as buffering agent, and 0.5 g EDTA, acting as chelating agent, to the solution, which was stirred for about 5 minutes. Then the solution was cooled to 30°C during stirring.

### Mixture 2

To a beaker containing 15.9 g ethanol of room temperature, acting as solvent, was added 0.045 g peppermint oil, acting as flavoring agent. The liquid was mixed for 2 minutes.

### Final mixture

Mixture 2 was added during stirring to a beaker containing 150 ml water. Gently 10 g nicotine (base) was added to the beaker. Then Mixture 1 was added to the beaker and stirred for 5 minutes. The pH of the Final mixture was checked and adjusted to about pH 6.0 with hydrochloric acid and to volume with water.

### Example 4

Manufacturing of a 1000 ml formulation with 17.5 mg nicotine/ml and around pH 9.0.

### Mixture 1

To a beaker containing 600 ml water of room temperature was added 12.0 g Synperonic^{®} PE/F27, being a poloxamer acting as non-ionic surface active agent. The additive was dissolved during stirring for about 20 minutes. Then was added 0.5 g EDTA, acting as chelating agent, and 0.4 g sodium saccharin, acting as sweetener, to the liquid which was stirred until all ingredients were dissolved. Then was added 16.8 g sodium hydrogen carbonate, acting as buffering agent, and the solution was stirred until a clear solution was obtained.

### Mixture 2

To a beaker containing 250.0 g ethanol of room temperature, acting as solvent, was added 0.7 g methyl para-hydroxybenzoate acting as preservative, and 0.3 g propyl para-hydroxybenzoate acting as preservative. The liquid was mixed until the ingredients were dissolved. Then was added 5.0 g peppermint oil, acting as flavoring agent, and 1.5 g aroma agent. The liquid was mixed until a clear solution was obtained.

### Final mixture

Mixture 2 was gently added to Mixture 1 during stirring for about 1 minute. Then was added 17.5 g nicotine (base) and the liquid was stirred for about 2 minutes. The pH of the Final mixture was checked and adjusted to around pH 9.0 with hydrochloric acid. The Final mixture was transferred to a 1000 ml volumetric flask and adjusted to 1000 ml volume by water. Finally the pH of the solution was checked to remain at around pH 9.0.

### Example 5

Manufacturing of a 1000 ml formulation with 14.3 mg nicotine/ml and around pH 9.0.

### Mixture 1

To a beaker containing 600 ml water of room temperature was added 20.0 g Synperonic^{®} PE/F27 being a poloxamer, acting as non-ionic surface active agent. The additive was dissolved during stirring for about 20 minutes. Then was added 2.0 g Acesulfame K, acting as sweetener, to the liquid which was stirred until all ingredients were dissolved. Then was added 20.0 g sodium hydrogen carbonate, acting as buffering agent, and the liquid was stirred until a clear solution was obtained.

### Mixture 2

To a beaker containing 95.0 g ethanol of room temperature, acting as solvent, was added 3.5 g peppermint oil, acting as flavoring agent, and 1.0 g aroma agent. The liquid was mixed until a clear solution was obtained.

### Final mixture

Mixture 2 was gently added to Mixture 1 during stirring for about 1 minute. Then was added 14.3 g nicotine (base) and the liquid was stirred for about 2 minutes. The pH of the Final mixture was checked and adjusted to around pH 9.0 with hydrochloric acid. The Final mixture was transferred to a 1000 ml volumetric flask and adjusted to 1000 ml volume by water. Finally the pH of the solution was checked to remain at around pH 9.0.

The formulation according to Example 5 is a preferred composition.

### Example 6

Manufacturing of a 1000 ml formulation with 14.3 mg nicotine/ml and around pH 9.0.

### Mixture 1

To a beaker containing 600 ml water of room temperature was added 20.0 g Synperonic^{®} PE/F27 being a poloxamer, acting as non-ionic surface active agent. The additive was dissolved during stirring for about 20 minutes. Then was added 0.2 g benzalkonium chloride, acting as preservative, and 2.0 g Acesulfame K, acting as sweetener, to the liquid which was stirred until all ingredients were dissolved. Then was added 20.0 g sodium hydrogen carbonate, acting as buffering agent, and the liquid was stirred until a clear solution was obtained.

### Mixture 2

To a beaker containing 95.0 g ethanol of room temperature, acting as solvent, was added 3.5 g peppermint oil, acting as flavoring agent, and 1.0 g aroma agent. The liquid was mixed until a clear solution was obtained.

### Final mixture

Mixture 2 was gently added to Mixture 1 during stirring for about 1 minute. Then was added 14.3 g nicotine (base) and the liquid was stirred for about 2 minutes. The pH of the Final mixture was checked and adjusted to around pH 9.0 with hydrochloric acid. The Final mixture was transferred to a 1000 ml volumetric flask and adjusted to 1000 ml volume by water. Finally the pH of the solution was checked to remain at around pH 9.0.

The formulation according to Example 6 is a another preferred composition.

### Example 7

Manufacturing of a 1000 ml formulation with 17.5 mg nicotine/ml and pH 10.94.

To a beaker containing 950 ml water of room temperature was added 17.5 g nicotine (base) during stirring for about 5 minutes. The volume was adjusted to 1000 ml volume by addition of water. Finally the pH was checked.

### Example 8

Manufacturing of a 1000 ml formulation with 17.5 mg nicotine/ml and pH 11.55.

To a beaker containing 950 ml water of room temperature was added 35 g sodium carbonate anhydrous during stirring until complete dissolution. Then 17.5 g nicotine-(base) was added during stirring for about 5 minutes. The volume was adjusted to 1000 ml volume by addition of water. Finally the pH was checked.

### Example 9

Manufacturing of a 1000 ml formulation with 15.65 mg nicotine/ml and pH 11.79.

To a beaker containing 950 ml water of room temperature was added 158 g glycine sodium salt during stirring until complete dissolution. Then 15.65 g nicotine (base) was added during stirring for about 5 minutes. The volume was adjusted to 1000 ml volume by addition of water. Finally the pH was checked.

### Example 10

### Buffer capacity determinations

**Method:** 10.0 ml of the respective below solutions was titrated with 0.1 M HCl to pH 7.0. The amount of 0.1 M HCl needed to decrease pH from 9.0 to 8.0 was determined.

### Definitions:

(1) Sodium hydrogen carbonate (NaHCO₃). Mw: 84.0
(2) Disodium phosphate dodecahydrate (Na₂HPO₄,12H₂O) Mw: 358.1

| **Ingredient\Batch** | DKN0293 | DKN0294 | DKN0295 | DKN0296 | DKN0290 | DKN0291 |
|---|---|---|---|---|---|---|
| Nicotine (mg/ml) | | | 10.0 | | 10.0 | 10.0 |
| NaHCO₃ (mg/ml) | 16.8 | | 16.8 | 8.4 | | |
| Na₂HPO₄,12H₂O (mg/ml) | | 71.6 | | 35.8 | 71.6 | |
| Purified water ad | 1 ml | 1 ml | 1 ml | 1 ml | 1 ml | 1 ml |
| Buffer Capacity PH=9.0-8.0 (mekv/l) | 26.5 | 9.5 | 50 | 15.8 | 40 | 29 |

All solutions were adjusted to a pH of 9.0 when needed. A higher pH may cause irritation and corrosion, which might be harmful to the tissue of the oral cavity.

| | |
|---|---|
| 16.8 mg/ml of | NaHCO₃ corresponds to 0.2 M. |
| 71.6 mg/ml of | Na₂HPO₄,12H₂O corresponds to 0.2 M. |
| 8.4 mg/ml of | NaHCO₃ corresponds to 0.1 M. |
| 35.8 mg/ml of | Na₂HPO₄,12H₂O corresponds to 0.1 M |

Nicotine base has an alkalizing effect, but has too weak a buffering capacity on its own. The buffering capacity of the formulation is significantly and sufficiently increased when a buffering agent is added.

The above data clearly show that the present formulations have a good buffering capacity, providing for the desired rapid transmucousal uptake of nicotine.

A liquid pharmaceutical formulation according to the present invention may be administered using suitable devices being available on the market, e g spray devices.

### Analysis of nicotine

The analysis of nicotine uptake and of the effect of the invention may be done according to standard procedures known in the art, e g using a bioanalysis for the determination of nicotine in the plasma of a subject.

### Effects of the invention

Comparative tests were conducted as described above under Legend of figures.

Figure 1 shows that with a liquid pharmaceutical formulation according to the present invention the venous blood plasma level of nicotine ascends signifycantly more rapidly than with Nicorette Microtab^{®}. Nicorette Microtab^{®} has the same pharmacokinetic profile as, i e is pharmacologically equivalent with, Nicorette Gum^{®} and all other nicotine chewing gums currently on the market. Nicotine chewing gums presently represent around half of the world sales of medicinal nicotine-containing products for smoking cessation and similar indications.

Figure 2 shows that the higher the pH of a liquid pharmaceutical formulation according to the present invention the faster the absorption kinetics and the higher the plasma concentration of nicotine.

Figures 3 and 4 further show that a formulation according to the present invention provides for a fast craving relief manifested through a significantly faster reduction in the urge to smoke compared to known oral nicotine formulations.

### Use for manufacturing

Nicotine in any form may be used for the manufacturing of a liquid pharmaceutical formulation according to the invention for the treatment of a disease or medical indication selected from the group consisting of reduction in use of tobacco, cessation of use of tobacco, other use of tobacco, temporary abstinence from abstaining from using tobacco, Alzheimer's disease, Crohn's disease, Parkinson's disease, Tourette's syndrome, and ulcerative colitis; and weight control.

## Claims

1. A liquid pharmaceutical formulation comprising nicotine, **characterized in that** the nicotine is present as nicotine base, **in that** it is for administration to the oral cavity by spraying, dropping or pipetting, preferably by spraying, most preferably by spraying under the tongue, **in that** it is alkalized by buffering and/or pH regulation in such a way that upon administration to a subject the pH of the liquid of the oral cavity of the subject is transiently increased by 0.3 to 4 pH units.

2. A liquid pharmaceutical formulation according to claim 1, **characterized in that** it is alkalized by buffering and/or pH regulation in such a way that upon administration to a subject the pH of the liquid of the oral cavity of the subject is transiently increased by 0.5 to 2.5 pH units.

3. A liquid pharmaceutical formulation according to any of claims 1 - 2, **characterized in that** it is alkalized by buffering and/or pH regulation by the use of one or more buffering agents selected from the group consisting of a carbonate, such as mono-carbonate, bicarbonate or sesquicarbonate; glycinate, phosphate, glycerophosphate, acetate, gluconate or citrate of an alkali metal, such as potassium or sodium, or of ammonium, and mixtures thereof; and/or by the use of pH regulating agents, such as agents selected from the group consisting of sodium hydroxide, potassium hydroxide, calcium hydroxide and calcium oxide; and/or by using at least partly pH regulating forms of nicotine.

4. A liquid pharmaceutical formulation according to any of claims 1 - 3, wherein the amount of nicotine base delivered at each incidence of administration is about 0.05 - 10 mg.

5. A liquid pharmaceutical formulation according to claim 4, wherein the amount of nicotine base delivered at each incidence of administration is about 0.25 - 6 mg.

6. A liquid pharmaceutical formulation according to claim 5, wherein the amount of nicotine base delivered at each incidence of administration is about 0.5 - 4 mg.

7. A liquid pharmaceutical formulation according to any of claims 1 - 6, wherein the liquid phase comprises water.

8. A liquid pharmaceutical formulation according to any of claims 1 - 6, wherein the liquid phase comprises an alcohol, such as ethanol, glycerol, propylene glycol and polyethylene glycol, or mixtures thereof.

9. A liquid pharmaceutical formulation according to any of claims 1 - 6, wherein the liquid phase comprises one or more lipids.

10. A liquid pharmaceutical formulation according to any of claims 1 - 9, wherein the liquid phase comprises water and/or an alcohol, such as ethanol, glycerol, propylene glycol and polyethylene glycol, and/or one or more lipids or mixtures thereof.

11. A liquid pharmaceutical formulation according to any claims 1-10, **characterized in that** it further comprises one or more flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of peppermint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary;
natural flavors and aroma agents including either diluted solutions of essential oils or concentrates of flavor components with natural origin from e g fruits, berries, nuts, spices, mints, tobacco, cocoa, coffee, tea, vanilla, liquorice, caramel, toffee, honey, wine, liquors and brews;
synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews;
and mixtures thereof.

12. A liquid pharmaceutical formulation according to any of claims 1 - 11, **characterized in that** it further comprises one or more stabilizing additives, such as those selected from the group consisting of antioxidants including vitamin E, i e tocopheroles, vitamin C, i e ascorbic acid and its salts, sodium pyrosulfite, butylhydroxytoluene, butylated hydroxyanisole; and preservatives including parabenes, benzalkonium chloride, chlorbutanol, benzyl alcohol, beta-phenylethyl alcohol, cetylpyridinium chloride; and chelating agents, such as EDTA; and galates, such as propyl galate.

13. A liquid pharmaceutical formulation according to any of claims 1 - 12, **characterized in that** it further comprises one or more additives selected from the group consisting of:
thickening agents, such as natural, semisynthetic or synthetic polymers, e g starch and starch derivatives, cellulose and cellulose derivatives, polyethylene glycols and derivatives thereof, polyacrylates, and polyvinyl esters and ethers;
enhancers, such as azone;
vitamins, such as vitamins C and E;
minerals, such as fluorides, especially sodium fluoride, sodium monofluoro phosphate and stannous fluoride;
anti-odours, such as zinc and cyclodextrins;
sweeteners including one or more synthetic sweetening agents and/or natural sugars, such as those selected from the groups consisting of
artificial sweeteners e g saccharin and its sodium and calcium salts, aspartame, acesulfame and its potassium salt, thaumatin and glycyrrhizin;
polyhydric alcohols such as sorbitol, xylitol, mannitol and glycerol; monosaccharides including glucose (also called dextrose), fructose (also called laevulose) and galactose;
disaccharides including saccharose (also called sucrose), lactose (also called milk sugar) and maltose (also called malt sugar);
mixtures of sugars including liquid glucose syrup e g starch hydrolysates containing a mixture of chiefly dextrose, maltose, dextrins and water, invert sugar syrup e g sucrose inverted by invertase containing a mixture of dextrose, laevulose and water, high sugar content syrups such as treacle, honey and malt extract;
and mixtures thereof;

14. A liquid pharmaceutical formulation according to any of claims 1 - 13, **characterized in that** one or more of the compounds of the liquid pharmaceutical formulation is/are solubilized in one or more surface active agents and/or emulsifiers, such as nonionic, cationic, anionic or zwitterionic surfactants, including amphiphilic block copolymers, or mixtures thereof.

15. A liquid pharmaceutical formulation according to claim 14, **characterized in that** one or more of the compounds of the liquid pharmaceutical formulation is/are solubilized in one or more surface active agents selected from
nonionic surface-active agents including poloxamers, e g
poly (oxypropylene)-poly(oxyethylene) block copolymers, polyoxyethylene alkyl ethers, polyoxyethylene castor oil derivatives, polyoxyethylene sorbitan fatty acid esters, mono- and diglycerides and esters thereof, polyoxyethylene stearates, polyglycerolesters of fatty acids (including polyglycerolpolyricinoleic acid (PGPR)), and sorbitan fatty acid esters,
cationic surface-active agents including secondary, quaternary and tertianary ammonium compounds and cationic phospholipids,
anionic surface-active agents including fatty acid salts, lactylates, especially sodium and/or calcium stearoyllactylate, alkyl sulphates, alkyl sulphonates, latanol, and anionic phospholipids, such as phosphatidylserine,
zwitterionic surface-active agents including zwitterionic phospholipids, such as phosphatidylcholine and phosphatidylethanolamine,
or mixtures thereof,
preferably surface-active agents or mixtures thereof being nonionic.

16. A liquid pharmaceutical formulation according to any of claims 1 - 15, **characterized in that** it comprises nicotine base, sodium hydrogen carbonate acting as buffering agent, water acting as solvent, ethanol acting as co-solvent, a poloxamer acting as surface active agent, EDTA acting as chelating agent, Acesulfame K acting as sweetener, optionally one or more preservatives, and optionally one or more flavoring or aroma agents.

17. A system for delivering nicotine to a subject, comprising a liquid pharma-ceutical formulation according to any of claims 1-16 and at least one other means for delivering nicotine to a subject.

18. A system for obtaining reduction of the urge to smoke or use of tobacco and/or for providing a sense of smoking satisfaction without smoking, comprising a liquid pharmaceutical formulation according to any of claims 1-16 and at least one other means for obtaining reduction of the urge to smoke or use tobacco.

19. A system according to any of claims 17 and 18, wherein the at least one other means is/are selected from the group consisting of administration through chewing gums, nasal sprays, transdermal patches, inhaling devices, lozenges, tablets and parenteral means or methods, subcutaneous means or methods, intravenous means or methods, rectal means or methods, vaginal means or methods and transmucosal means or methods; or use of tobacco.

20. A system according to claim 19, wherein the at least other means comprises administration of nicotine.

21. A method for manufacturing a liquid pharmaceutical formulation according to any of claims 1 - 16, comprising the steps of:
a) making a first mixture comprising a first solvent, at least one buffering agent and/or other means for pH regulation, and optionally nicotine base,
b) optionally adding first components to said first mixture, said first components optionally having been initially solubilized,
c) optionally making one or more second mixture(s) comprising one or more second solvent(s) and second component(s), which may comprise nicotine base,
d) optionally mixing the first mixture and the optional one or more second mixture(s) to a final mixture, optionally under addition of one or more further solvent(s), and optionally under addition of nicotine base,
e) optionally adjusting the pH of the final mixture.

22. A method according to claim 21, wherein the mixing is done in a temperature from about room temperature to about 95°C.

23. The method according to any of claims 21 and 22, wherein the buffering and/or pH regulation is obtained by the use of one or more buffering agents selected from the group consisting of a carbonate, such as monocarbonate, bicarbonate or sesquicarbonate; glycinate, phosphate, glycerophosphate, acetate, gluconate or citrate of an alkali metal, such as potassium or sodium, or ammonium, and mixtures thereof; and/or by the use of pH regulating agents, such as agents selected from the group consisting of sodium hydroxide, potassium hydroxide, calcium hydroxide and calcium oxide; and/or by using at least partly pH regulating forms of nicotine.

24. The method according to any of claims 21 - 23, wherein the first solvent and the optional one or more second solvent(s) is/are chosen from water and/or an alcohol, such as ethanol, glycerol, propylene glycol and polyethylene glycol, and/or one or more lipids or mixtures thereof.

25. The method according to any of claims 21-24, wherein the optional first and second components are chosen from
one or more flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of peppermint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary;
natural flavors and aroma agents including either diluted solutions of essential oils or concentrates of flavor components with natural origin from e g fruits, berries, nuts, spices, mints, tobacco, cocoa, coffee, tea, vanilla, liquorice, caramel, toffee, honey, wine, liquors and brews;
synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews;
and mixtures thereof;
one or more stabilizing additives, such as those selected from the group consisting of antioxidants including vitamin E, i e tocopheroles, vitamin C, i e ascorbic acid and its salts, sodium pyrosulfite, butylhydroxytoluene, butylated hydroxyanisole; and preservatives including parabenes, benzalkonium chloride, chlorbutanol, benzyl alcohol, beta-phenylethylal alcohol, cetylpyridinium chloride; and chelating agents, such as EDTA; and galates, such as propyl galate;
one or more additives selected from the group consisting of
thickening agents, such as natural, semisynthetic or synthetic polymers, e g starch and starch derivatives, cellulose and cellulose derivatives, polyethylene glycols and derivatives thereof, polyacrylates, and polyvinyl esters and ethers;
enhancers, such as azone;
vitamins, such as vitamins C and E;
minerals, such as fluorides, especially sodium fluoride, sodium monofluoro phosphate and stannous fluoride;
anti-odours, such as zinc and cyclodextrins;
sweeteners including one or more synthetic sweetening agents and/or natural sugars, such as those selected from the groups consisting of
artificial sweeteners e g saccharin and its sodium and calcium salts, aspartame, acesulfame and its potassium salt, thaumatin and glycyrrhizin;
polyhydric alcohols such as sorbitol, xylitol, mannitol and glycerol; monosaccharides including glucose (also called dextrose), fructose (also called laevulose) and galactose;
disaccharides including saccharose (also called sucrose), lactose (also called milk sugar) and maltose (also called malt sugar);
mixtures of sugars including liquid glucose syrup e g starch hydrolysates containing a mixture of chiefly dextrose, maltose, dextrins and water, invert sugar syrup e g sucrose inverted by invertase containing a mixture of dextrose, laevulose and water, high sugar content syrups such as treacle, honey and malt extract;
and mixtures thereof.

26. The method according to any of claims 21 - 25, wherein the first and/or second components is/are solubilized in one or more surface active agents and/or emulsifiers, such as nonionic, cationic, anionic or zwitterionic surfactants, including amphiphilic block copolymers, or mixtures thereof, whereby preferably the one or more surface active agents is/are selected from
nonionic surface-active agents including poloxamers, e g poly(oxypropylene)-poly(oxyethylene) block copolymers, polyoxyethylene alkyl ethers, polyoxyethylene castor oil derivatives, polyoxyethylene sorbitan fatty acid esters, mono- and diglycerides and esters thereof, polyoxyethylene stearates, polyglycerolesters of fatty acids (including polyglycerolpolyricinoleic acid (PGPR)), and sorbitan fatty acid esters,
cationic surface-active agents including secondary, quaternary and tertianary ammonium compounds and cationic phospholipids,
anionic surface-active agents including fatty acid salts, lactylates, especially sodium and/or calcium stearoyllactylate, alkyl sulphates, alkyl sulphonates, latanol, and anionic phospholipids, such as phosphatidylserine,
zwitterionic surface-active agents including zwitterionic phospholipids, such as phosphatidylcholine and phosphatidylethanolamine,
or mixtures thereof, whereby most preferably the surface-active agents or mixtures thereof are nonionic.

27. A liquid pharmaceutical formulation according to any of claims 1 - 16 for use in therapy.

28. A liquid pharmaceutical formulation according to claim 27, wherein the therapy is treatment of a disease selected from the group consisting of addiction to tobacco or nicotine, Alzheimer's disease, Crohn's disease, Parkinson's disease, Tourette's syndrome, ulcerative colitis; and weight control.

29. Use of nicotine base for the manufacturing of a liquid pharmaceutical formulation according to any of claims 1 - 16 for the treatment of a disease selected from the group consisting of addiction to tobacco or nicotine, Alzheimer's disease, Crohn's disease, Parkinson's disease, Tourette's syndrome, ulcerative colitis; and weight control.

## Patentansprüche

1. Flüssige pharmazeutische Formulierung, umfassend Nikotin, **dadurch gekennzeichnet, dass** das Nikotin als Nikotin-Base vorliegt, dass diese für eine Verabreichung in die Mundhöhle ist durch Sprühen, Tropfen oder Pipettieren, vorzugsweise durch Sprühen, besonders bevorzugt durch Sprühen unter die Zunge, dass es so alkalisiert ist durch Puffern und/oder pH-Einstellung, dass nach Verabreichung an ein Subjekt der pH der Flüssigkeit der Mundhöhle des Subjekts sich vorübergehend um 0,3 bis 4 pH-Einheiten erhöht.

2. Flüssige pharmazeutische Formulierung gemäß Anspruch 1, **dadurch gekennzeichnet, dass** diese so alkalisiert ist durch Puffern und/oder pH-Einstellung, dass nach Verabreichen an ein Subjekt der pH der Flüssigkeit der Mundhöhle des Subjekts sich vorübergehend um 0,5 bis 2,5 pH-Einheiten erhöht.

3. Flüssige pharmazeutische Formulierung gemäß einem der Ansprüche 1-2, **dadurch gekennzeichnet, dass** diese alkalisiert ist durch Puffern und/oder pH-Einstellung unter Verwendung eines oder mehrerer Puffermittel ausgewählt aus der Gruppe bestehend aus einem Carbonat, wie Monocarbonat, Bicarbonat oder Sesquicarbonat; Glycinat, Phosphat, Glycerophosphat, Acetat, Gluconat oder Citrat eines Alkalimetalls, wie Kalium oder Natrium, oder von Ammonium, und Mischungen davon; und/oder durch Verwendung von pH-Einstellmitteln, wie aus der Gruppe bestehend aus Natriumhydroxid, Kaliumhydroxid, Calciumhydroxid und Calciumoxid ausgewählten Mitteln; und/oder durch Verwenden wenigstens teilweise pHeinstellenden Formen von Nikotin.

4. Flüssige pharmazeutische Formulierung gemäß einem der Ansprüche 1-3, wobei die Menge an Nikotin-Base, die bei jedem Verabreichungsereignis zugeführt wird, etwa 0,05-10 mg beträgt.

5. Flüssige pharmazeutische Formulierung gemäß Anspruch 4, wobei die Menge an Nikotin-Base, die bei jedem Verabreichungsereignis zugeführt wird, etwa 0,25-6 mg beträgt.

6. Flüssige pharmazeutische Formulierung gemäß Anspruch 5, wobei die Menge an Nikotin-Base, die bei jedem Verabreichungsereignis zugeführt wird, etwa 0,5-4 mg beträgt.

7. Flüssige pharmazeutische Formulierung gemäß einem der Ansprüche 1-6, wobei die flüssige Phase Wasser umfasst.

8. Flüssige pharmazeutische Formulierung gemäß einem der Ansprüche 1-6, wobei die flüssige Phase einen Alkohol, wie Ethanol, Glycerol, Propylenglykol und Polyethylenglykol, oder Mischungen davon umfasst.

9. Flüssige pharmazeutische Formulierung gemäß einem der Ansprüche 1-6, wobei die flüssige Phase ein oder mehrere Lipide umfasst.

10. Flüssige pharmazeutische Formulierung gemäß einem der Ansprüche 1-9, wobei die flüssige Phase Wasser und/oder einen Alkohol, wie Ethanol, Glycerol, Propylenglykol und Polyethylenglykol, und/oder ein oder mehrere Lipide oder Mischungen davon umfasst.

11. Flüssige pharmazeutische Formulierung gemäß einem der Ansprüche 1-10, **dadurch gekennzeichnet, dass** diese ferner einen oder mehrere Geschmacks- und/oder Aromastoffe, wie diejenigen, die ausgewählt sind aus der Gruppe bestehend aus ätherischen Ölen, erhalten durch Destillationen, Lösungsmittelextraktionen oder Kaltpressungen von frischen oder getrockneten Blüten, Knospen, Blättern, Stielen, Früchten, Samen, Schale, Rinde oder Wurzel, z.B. ein Öl von Pfefferminze, grüner Minze, Eukalyptus, Wintergrün, Niaouli, Nelke, Kardamom, Zimtbaum, Bittermandel, Koreander, Kümmel, Ingwer, Wacholder, Orange, Pomeranze, Zitrone, Grapefruit, Mandarine, Bergamotte, Thymian, Fenchel und Rosmarin;
natürliche Geschmacksstoffe und Aromastoffe, umfassend entweder verdünnte Lösungen von ätherischen Ölen oder Konzentrate von Geschmackskomponenten mit natürlichem Ursprung aus beispielsweise Früchten, Beeren, Nüssen, Gewürzen, Minze, Tabak, Kakao, Kaffee, Tee, Vanille, Lakritze, Karamell, Toffee, Honig, Wein, Spirituosen und Braugetränken;
synthetische Geschmacksstoffe und Aromastoffe, bestehend aus Mischungen von Chemikalien, umfassend Kohlenwasserstoffe, Alkohole, Aldehyde, Ester, Ketone, Ether und Oxide, die gemischt sind, um dem natürlichen Geschmack von beispielsweise Früchten, Beeren, Nüssen, Gewürzen, Minze, Tabak, Kakao, Kaffee, Tee, Vanille, Lakritze, Karamell, Toffee, Honig, Wein, Spirituosen oder Braugetränken zu entsprechen;
und Mischungen davon umfassen.

12. Flüssige pharmazeutische Formulierung gemäß einem der Ansprüche 1-11, **dadurch gekennzeichnet, dass** diese ferner ein oder mehrere Stabilisiermittel, wie diejenigen, die ausgewählt sind aus der Gruppe bestehend aus Antioxidationsmitteln, einschließlich Vitamin E, d.h. Tocopherolen, Vitamin C, d.h. Ascorbinsäure und deren Salzen, Natriumpyrosulfit, Butylhydroxytoluol, butyliertem Hydroxyanisol; und Konservierungsstoffe, einschließlich Parabene, Benzalkoniumchlorid, Chlorbutanol, Benzylalkohol, beta-Phenylethylalkohol, Cetylpyridiniumchlorid; und Komplexbildner, wie EDTA; und Galate, wie Propylgalat, umfasst.

13. Flüssige pharmazeutische Formulierung gemäß einem der Ansprüche 1-12, **dadurch gekennzeichnet, dass** diese ferner ein oder mehrere Additive umfasst, die ausgewählt sind aus der Gruppe bestehend aus:
Verdickungsmitteln, wie natürlichen, semisynthetischen oder synthetischen Polymeren, z.B. Stärke und Stärkederivaten, Cellulose und Cellulosederivaten, Polyethylenglykolen und Derivaten davon, Polyacrylaten, und Polyvinylestern und Ethern;
Geschmacksverstärkern, wie Azon;
Vitaminen, wie den Vitaminen C und E;
Mineralstoffen, wie Fluoriden, insbesondere Natriumfluorid, Natriummonofluorphosphat und Zinndifluorid;
Geruchsentfernern, wie Zink und Cyclodextrinen;
Süßstoffen, einschließlich einem oder mehreren synthetischen Süßungsmitteln und/oder natürlichen Zuckern, wie denjenigen, die aus der Gruppe ausgewählt sind bestehend aus
künstlichen Süßstoffen, z.B. Saccharin und dessen Natrium- und Calciumsalzen, Aspartam, Acesulfam und dessen Kaliumsalz, Thaumatin und Glycyrrhizin;
mehrwertigen Alkoholen, wie Sorbitol, Xylitol, Mannitol und Glycerol;
Monosacchariden, einschließlich Glucose (auch Dextrose genannt), Fructose (auch Lävulose genannt) und Galactose;
Disacchariden, einschließlich Saccharose (auch Sucrose genannt), Lactose (auch Milchzucker genannt) und Maltose (auch Malzzucker genannt);
Mischungen von Zuckern, einschließlich flüssigem Glucosesirup, z.B. Stärkehydrolysaten, enthaltend eine Mischung von hauptsächlich Dextrose, Maltose, Dextrinen und Wasser, Invertzuckersirup, z.B. durch Invertase invertierte Sucrose, enthaltend eine Mischung von Dextrose, Lävulose und Wasser, Sirupe mit hohem Zuckergehalt, wie Melasse, Honig und Malzextrakt;
und Mischungen davon.

14. Flüssige pharmazeutische Formulierung gemäß einem der Ansprüche 1-13, **dadurch gekennzeichnet, dass** eine oder mehrere Verbindungen der flüssigen pharmazeutischen Formulierung solubilisiert ist/sind in einem oder mehreren oberflächenaktiven Mitteln und/oder Emulgatoren, wie nicht-ionischen, kationischen, anionischen oder zwitterionischen oberflächenaktiven Mitteln, einschließlich amphiphilen Blockcopolymeren, oder Mischungen davon.

15. Flüssige pharmazeutische Formulierung gemäß Anspruch 14, **dadurch gekennzeichnet, dass** eine oder mehrere der Verbindungen der flüssigen pharmazeutischen Formulierung solubilisiert ist/sind in einem oder mehreren oberflächenaktiven Mitteln ausgewählt aus
nicht-ionischen oberflächenaktiven Mitteln, einschließlich Poloxameren, z.B. Poly(oxypropylen)-Poly(oxyethylen)-Blockcopolymeren, Polyoxyethylenalkylethern, Polyoxyethylen-Kastoröl-Derivaten, Polyoxyethylensorbitanfettsäureestern, Mono- und Diglyceriden und Estern davon, Polyoxyethylenstearaten, Polyglycerolestern von Fettsäuren (einschließlich Polyglycerolpolyricinolsäure (PGPR)), und Sorbitanfettsäureestem,
kationischen oberflächenaktiven Mitteln, einschließlich sekundären, quaternären und tertiären Ammoniumverbindungen und kationischen Phospholipiden,
anionischen oberflächenaktiven Mitteln, einschließlich Fettsäuresalzen, Lactylaten, insbesondere Natrium- und/oder Calciumstearoyllactylat, Alkylsulfaten, Alkylsulfonaten, Lactanol und anionischen Phospholipiden, wie Phosphatidylserin,
zwitterionischen oberflächenaktiven Mitteln, einschließlich zwitterionischen Phospholipiden, wie Phosphatidylchloin und Phosphatidylethanolamin,
oder Mischungen davon,
wobei die oberflächenaktiven Mittel oder Mischungen davon vorzugsweise nicht-ionisch sind.

16. Flüssige pharmazeutische Formulierung gemäß einem der Ansprüche 1-15, **dadurch gekennzeichnet, dass** diese Nikotin-Base, als Puffermittel dienendes Natriumhydrogencarbonat, als Lösungsmittel dienendes Wasser, als Verschnittmittel dienendes Ethanol, ein als oberflächenaktives Mittel dienendes Poloxamer, als Komplexbildner dienendes EDTA, als Süßstoff dienendes Acesulfam K, gegebenenfalls ein oder mehrere Konservierungsstoffe und gegebenenfalls ein oder mehrere Geschmacks- oder Aromastoffe umfasst.

17. System für die Zufuhr von Nikotin an ein Subjekt, umfassend eine flüssige pharmazeutische Formulierung gemäß einem der Ansprüche 1-16 und wenigstens ein anderes Mittel zum Zuführen von Nikotin an ein Subjekt.

18. System zum Erzielen einer Verringerung des Verlangens zu rauchen oder Tabak zu verwenden und/oder zum Bereitstellen eines Gefühls der Raucherbefriedigung ohne Rauchen, umfassend eine flüssige pharmazeutische Formulierung gemäß einem der Ansprüche 1-16 und wenigstens ein anderes Mittel zum Erzielen einer Verringerung des Verlangens zu rauchen oder Tabak zu verwenden.

19. System gemäß einem der Ansprüche 17 und 18, wobei das wenigstens eine andere Mittel ausgewählt ist/sind aus der Gruppe bestehend aus einer Verabreichung mittels Kaugummis, Nasensprays, transdermalen Pflastern, Inhaliervorrichtungen, Lutschtabletten, Tabletten und parenteralen Mitteln oder Verfahren, subkutanen Mitteln oder Verfahren, intravenösen Mitteln oder Verfahren, rektalen Mitteln oder Verfahren, vaginalen Mitteln oder Verfahren und transmucosalen Mitteln oder Verfahren; oder Verwendung von Tabak.

20. System gemäß Anspruch 19, wobei das wenigstens eine andere Mittel eine Verabreichung von Nikotin umfasst.

21. Verfahren zur Herstellung einer flüssigen pharmazeutischen Formulierung gemäß einem der Ansprüche 1-16, umfassend die Schritte:
a) Herstellen einer ersten Mischung, umfassend ein erstes Lösungsmittel, wenigstens ein Puffermittel und/oder andere Mittel zur pH-Einstellung und gegebenenfalls Nikotin-Base,
b) gegebenenfalls Zugeben von ersten Komponenten zu der ersten Mischung, wobei die ersten Komponenten gegebenenfalls zunächst solubilisiert worden sind,
c) gegebenenfalls Herstellen einer oder mehrerer zweiten Mischung(en), umfassend ein oder mehrere zweite Lösungsmittel und eine zweite Komponente (zweite Komponenten), die Nikotin-Base umfassen können,
d) gegebenenfalls Mischen der ersten Mischung und der optionalen einen oder mehreren zweiten Mischung(en) zu einer Endmischung, gegebenenfalls unter Zugabe eines weiteren oder mehrerer weiterer Lösungmittel, und gegebenenfalls unter Zugabe von Nikotin-Base,
e) gegebenenfalls Einstellen des pH's der Endmischung.

22. Verfahren gemäß Anspruch 21, wobei das Mischen bei einer Temperatur von etwa Raumtemperatur bis etwa 95°C erfolgt.

23. Verfahren gemäß einem der Ansprüche 21 und 22, wobei das Puffern und/oder die pH-Einstellung erzielt wird durch Verwendung eines oder mehrerer Puffermittel ausgewählt aus der Gruppe bestehend aus einem Carbonat, wie Monocarbonat, Bicarbonat oder Sesquicarbonat; Glycinat, Phosphat, Glycerophosphat, Acetat, Gluconat oder Citrat eines Alkalimetalls, wie Kalium oder Natrium, oder Ammonium, und Mischungen davon; und/oder durch Verwendung von pH-Einstellmitteln, wie aus der Gruppe bestehend aus Natriumhydroxid, Kaliumhydroxid, Calciumhydroxid und Calciumoxid ausgewählten Mitteln; und/oder durch Verwenden wenigstens teilweise pH-einstellender Formen von Nikotin.

24. Verfahren gemäß einem der Ansprüche 21-23, wobei das erste Lösungsmittel und das optionale eine oder mehrere zweite Lösungsmittel ausgewählt ist/sind aus Wasser und/oder einem Alkohol, wie Ethanol, Glycerol, Propylenglycol und Polyethylenglycol, und/oder einem oder mehreren Lipiden oder Mischungen davon.

25. Verfahren gemäß einem der Ansprüche 21-24, wobei die optionalen ersten und zweiten Komponenten ausgewählt sind aus
einem oder mehreren Geschmacks- und/oder Aromastoffen, wie diejenigen, die ausgewählt sind aus der Gruppe bestehend aus ätherischen Ölen, erhalten durch Destillationen, Lösungsmittelextraktionen oder Kaltpressungen von frischen oder getrockneten Blüten, Knospen, Blättern, Stielen, Früchten, Samen, Schale, Rinde oder Wurzel, z.B. ein Öl von Pfefferminze, grüner Minze, Eukalyptus, Wintergrün, Niaouli, Nelke, Kardamom, Zimtbaum, Bittermandel, Koreander, Kümmel, Ingwer, Wacholder, Orange, Pomeranze, Zitrone, Grapefruit, Mandarine, Bergamotte, Thymian, Fenchel und Rosmarin;
natürlichen Geschmacksstoffen und Aromastoffen, umfassend entweder verdünnte Lösungen von ätherischen Ölen oder Konzentrate von Geschmackskomponenten mit natürlichem Ursprung aus beispielsweise Früchten, Beeren, Nüssen, Gewürzen, Minze, Tabak, Kakao, Kaffee, Tee, Vanille, Lakritze, Karamell, Toffee, Honig, Wein, Spirituosen und Braugetränken;
synthetischen Geschmacksstoffen und Aromastoffen, bestehend aus Mischungen von Chemikalien, umfassend Kohlenwasserstoffe, Alkohole, Aldehyde, Ester, Ketone, Ether und Oxide, die gemischt sind, um dem natürlichen Geschmack von beispielsweise Früchten, Beeren, Nüssen, Gewürzen, Minze, Tabak, Kakao, Kaffee, Tee, Vanille, Lakritze, Karamell, Toffee, Honig, Wein, Spirituosen oder Braugetränken zu entsprechen;
und Mischungen davon;
einem oder mehreren Stabilisieradditiven, wie denjenigen, die ausgewählt sind aus der Gruppe bestehend aus Antioxidationsmitteln, einschließlich Vitamin E, d.h. Tocopherolen, Vitamin C, d.h. Ascorbinsäure und deren Salze, Natriumpyrosulfit, Butylhydroxytoluol, butyliertem Hydroxyanisol; und Konservierungsstoffen, einschließlich Parabenen, Benzalkoniumchlorid, Chlorbutanol, Benzylalkohol, beta-Phenylethylalkohol, Cetylpyridiniumchlorid; und Komplexbildnern, wie EDTA; und Galaten, wie Propylgalat;
einem oder mehreren Additiven ausgewählt aus der Gruppe bestehend aus Verdickungsmitteln, wie natürlichen, semisynthetischen oder synthetischen Polymeren, z.B. Stärke und Stärkederivaten, Cellulose und Cellulosederivaten, Polyethylenglykolen und Derivaten davon, Polyacrylaten, und Polyvinylestern und Ethern;
Geschmacksverstärkern, wie Azon;
Vitaminen, wie den Vitaminen C und E;
Mineralstoffen, wie Fluoriden, insbesondere Natriumfluorid, Natriummonofluorphosphat und Zinndifluorid;
Geruchsentfernern, wie Zink und Cyclodextrinen;
Süßstoffen, einschließlich einem oder mehreren synthetischen Süßungsmitteln und/oder natürlichen Zuckern, wie denjenigen, die aus den Gruppen ausgewählt sind bestehend aus
künstlichen Süßstoffen, z.B. Saccharin und dessen Natrium- und Calciumsalzen, Aspartam, Acesulfam und dessen Kaliumsalz, Thaumatin und Glycyrrhizin;
mehrwertigen Alkoholen, wie Sorbitol, Xylitol, Mannitol und Glycerol;
Monosacchariden, einschließlich Glucose (auch Dextrose genannt), Fructose (auch Lävulose genannt) und Galactose;
Disacchariden, einschließlich Saccharose (auch Sucrose genannt), Lactose (auch Milchzucker genannt) und Maltose (auch Malzzucker genannt);
Mischungen von Zuckern, einschließlich flüssigem Glucosesirup, z.B. Stärkehydrolysaten, enthaltend eine Mischung von hauptsächlich Dextrose, Maltose, Dextrinen und Wasser, Invertzuckersirup, z.B. durch Invertase invertierte Sucrose, enthaltend eine Mischung von Dextrose, Lävulose und Wasser, Sirupe mit hohem Zuckergehalt, wie Melasse, Honig und Malzextrakt;
und Mischungen davon.

26. Verfahren gemäß einem der Ansprüche 21-25, wobei die erste und/oder zweite Komponente solubilisiert ist/sind in einem oder mehreren oberflächenaktiven Mitteln und/oder Emulgatoren, wie nicht-ionischen, kationischen, anionischen oder zwitterionischen oberflächenaktiven Mitteln, einschließlich amphiphilen Blockcopolymeren, oder Mischungen davon, wobei das eine oder mehrere oberflächenaktive Mittel vorzugsweise ausgewählt ist/sind aus
nicht-ionischen oberflächenaktiven Mitteln, einschließlich Poloxameren, z.B. Poly(oxypropylen)-Poly(oxyethylen)-Blockcopolymeren, Polyoxyethylenalkylethern, Polyoxyethylen-Kastoröl-Derivaten, Polyoxyethylensorbitanfettsäureestern, Mono- und Diglyceriden und Estern davon, Polyoxyethylenstearaten, Polyglycerolestern von Fettsäuren (einschließlich Polyglycerolpolyricinolsäure (PGPR)), und Sorbitanfettsäureestem,
kationischen oberflächenaktiven Mitteln, einschließlich sekundären, quaternären und tertiären Ammoniumverbindungen und kationischen Phospholipiden,
anionischen oberflächenaktiven Mitteln, einschließlich Fettsäuresalzen, Lactylaten, insbesondere Natrium- und/oder Calciumstearoyllactylat, Alkylsulfaten, Alkylsulfonaten, Lactanol und anionischen Phospholipiden, wie Phosphatidylserin,
zwitterionischen oberflächenaktiven Mitteln, einschließlich zwitterionischen Phospholipiden, wie Phosphatidylchloin und Phosphatidylethanolamin,
oder Mischungen davon,
wobei die oberflächenaktiven Mittel oder Mischungen davon vorzugsweise nicht-ionisch sind.

27. Flüssige pharmazeutische Formulierung gemäß einem der Ansprüche 1-16 zur Verwendung in der Therapie.

28. Flüssige pharmazeutische Formulierung gemäß Anspruch 27, wobei die Therapie eine Behandlung einer Krankheit ist, die aus der Gruppe bestehend aus Tabak- oder Nikotinsucht, Alzheimer-Krankheit, Crohn-Krankheit, Parkinson-Krankheit, Tourette-Syndrom, Colitis ulcerosa ausgewählt ist; und Gewichtskontrolle.

29. Verwendung von Nikotin-Base zur Herstellung einer flüssigen pharmazeutischen Formulierung gemäß einem der Ansprüche 1-16 zur Behandlung einer aus der Gruppe bestehend aus Tabak- oder Nikotinsucht, Alzheimer-Krankheit, Crohn-Krankheit, Parkinson-Krankheit, Tourette-Syndrom, Colitis ulcerosa ausgewählten Krankheit; und Gewichtskontrolle.

## Revendications

1. Préparation pharmaceutique liquide comprenant de la nicotine, **caractérisée en ce que** la nicotine est présente sous forme de nicotine base, **en ce qu'**elle est destinée à être administrée dans la cavité orale par pulvérisation, goutte à goutte ou pipetage, de préférence par pulvérisation, de manière préférée entre toutes par pulvérisation sous la langue, **en ce qu'**elle est alcalinisée par tamponnage et/ou régulation du pH de manière à ce que lors de l'administration à un sujet le pH du liquide de la cavité orale du sujet augmente de manière transitoire de 0,3 à 4 unités de pH.

2. Préparation pharmaceutique liquide selon la revendication 1, **caractérisée en ce qu'**elle est alcalinisée par tamponnage et/ou régulation du pH de manière à ce que lors de l'administration à un sujet le pH du liquide de la cavité orale du sujet augmente de manière transitoire de 0,5 à 2,5 unités de pH.

3. Préparation pharmaceutique liquide selon l'une quelconque des revendications 1 à 2, **caractérisée en ce qu'**elle est alcalinisée par tamponnage et/ou régulation du pH au moyen d'un ou de plusieurs agents tampons choisis dans le groupe constitué par un carbonate, tel qu'un monocarbonate, un bicarbonate ou un sesquicarbonate ; un glycinate, un phosphate, un glycérophosphate, un acétate, un gluconate ou un citrate d'un métal alcalin, tel que le potassium ou le sodium, ou d'ammonium, et leurs mélanges ; et/ou au moyen d'agents régulant le pH, tels que les agents choisis dans le groupe constitué par l'hydroxyde de sodium, l'hydroxyde de potassium, l'hydroxyde de calcium et l'oxyde de calcium ; et/ou en utilisant au moins partiellement des formes de nicotine régulant le pH.

4. Préparation pharmaceutique liquide selon l'une quelconque des revendications 1 à 3, dans laquelle la quantité de nicotine base délivrée à chaque administration est d'environ 0,05 mg à 10 mg.

5. Préparation pharmaceutique liquide selon la revendication 4, dans laquelle la quantité de nicotine base délivrée à chaque administration est d'environ 0,25 mg à 6 mg.

6. Préparation pharmaceutique liquide selon la revendication 5, dans laquelle la quantité de nicotine base délivrée à chaque administration est d'environ 0,5 mg à 4 mg.

7. Préparation pharmaceutique liquide selon l'une quelconque des revendications 1 à 6, dans laquelle la phase liquide comprend de l'eau.

8. Préparation pharmaceutique liquide selon l'une quelconque des revendications 1 à 6, dans laquelle la phase liquide comprend un alcool, tel que l'éthanol, le glycérol, le propylène glycol et le polyéthylène glycol, ou leurs mélanges.

9. Préparation pharmaceutique liquide selon l'une quelconque des revendications 1 à 6, dans laquelle la phase liquide comprend un ou plusieurs lipides.

10. Préparation pharmaceutique liquide selon l'une quelconque des revendications 1 à 9, dans laquelle la phase liquide comprend de l'eau et/ou un alcool, tel que l'éthanol, le glycérol, le propylène glycol et le polyéthylène glycol, et/ou un ou plusieurs lipides ou leurs mélanges.

11. Préparation pharmaceutique liquide selon l'une quelconque des revendications 1 à 10, **caractérisée en ce qu'**elle comprend en outre un ou plusieurs agents de saveur et/ou aromatisants, tels que ceux choisis dans le groupe constitué par les huiles essentielles obtenues par distillations, extractions par solvant ou expressions à froid de fleurs, de bourgeons, de feuilles, de tiges, de fruits, de graines, de pelures, d'écorces ou de racines à l'état frais ou sec, par exemple l'huile de menthe poivrée, de menthe verte, d'eucalyptus, de gaulthérie, de niaouli, de clou de girofle, de cardamome, de cannelle, d'amande amère, de coriandre, de carvi, de gingembre, de genièvre, d'orange, d'orange amère, de citron, de pamplemousse, de mandarine, de bergamote, de thym, de fenouil et de romarin ;
les agents de saveur et aromatiques naturels comprenant les solutions diluées d'huiles essentielles ou les concentrés de composants de saveur d'origine naturelle provenant, par exemple, de fruits, de baies, de noix, d'épices, de menthes, de tabac, de cacao, de café, de thé, de vanille, de réglisse, de caramel, de caramel anglais, de miel, de vin, de liqueurs et de décoctions ;
les agents de saveur et aromatiques synthétiques constitués de mélanges de produits chimiques comprenant des hydrocarbures, des alcools, des aldéhydes, des esters, des cétones, des éthers et des oxydes mélangés pour correspondre à la saveur naturelle, par exemple, de fruits, de baies, de noix, d'épices, de menthes, de tabac, de cacao, de café, de thé, de vanille, de réglisse, de caramel, de caramel anglais, de miel, de vin, de liqueurs ou de décoctions ;
et leurs mélanges.

12. Préparation pharmaceutique liquide selon l'une quelconque des revendications 1 à 11, **caractérisée en ce qu'**elle comprend en outre un ou plusieurs additifs stabilisants, tels que ceux choisis dans le groupe constitué par les antioxydants, comme la vitamine E, c'est-à-dire les tocophérols, la vitamine C, c'est-à-dire l'acide ascorbique et ses sels, le pyrosulfite de sodium, le butylhydroxytoluène, l'hydroxyanisole butylé ; et les conservateurs, comme les parabènes, le chlorure de benzalkonium, le chlorobutanol, l'alcool benzylique, l'alcool bêta-phényléthylique, le chlorure de cétylpyridinium ; et les agents de chélation, comme l'EDTA ; et les galates, comme le galate de propyle.

13. Préparation pharmaceutique liquide selon l'une quelconque des revendications 1 à 12**, caractérisée en ce qu'**elle comprend en outre un ou plusieurs additifs choisis dans le groupe constitué par :
les agents épaississants, comme les polymères naturels, semi-synthétiques ou synthétiques, par exemple l'amidon et les dérivés d'amidon, la cellulose et les dérivés de cellulose, les polyéthylène glycols et leurs dérivés, les polyacrylates et les esters et éthers de polyvinyle ;
les exhausteurs, comme l'azone ;
les vitamines, comme les vitamines C et E ;
les minéraux, comme les fluorures, en particulier le fluorure de sodium, le monofluorophosphate de sodium et le fluorure stanneux ;
les anti-odeurs, comme le zinc et les cyclodextrines ;
les édulcorants, comme un ou plusieurs agents édulcorants synthétiques et/ou sucres naturels, tels que ceux choisis dans le groupe constitué par
les édulcorants artificiels, par exemple la saccharine et ses sels de sodium et de calcium, l'aspartame, l'acésulfame et son sel de potassium, la thaumatine et la glycyrrhizine ;
les alcools polyhydriques, tels que le sorbitol, le xylitol, le mannitol et le glycérol ; les monosaccharides, comme le glucose (également appelé dextrose), le fructose (également appelé lévulose) et le galactose ;
les disaccharides, comme le saccharose (également appelé sucrose), le lactose (également appelé sucre de lait) et le maltose (également appelé sucre de malt) ;
les mélanges de sucre, comme le sirop de glucose liquide, par exemple les hydrolysats d'amidon contenant un mélange composé principalement de dextrose, de maltose, de dextrines et d'eau, le sirop de sucre inverti, par exemple le saccharose inverti par une invertase contenant un mélange de dextrose, de lévulose et d'eau, les sirops à teneur élevée en sucre, tels que la mélasse, le miel et un extrait de malt ;
et leurs mélanges.

14. Préparation pharmaceutique liquide selon l'une quelconque des revendications 1 à 13, **caractérisée en ce qu'**un ou plusieurs des composés de la préparation pharmaceutique liquide est/sont solubilisé(s) dans un ou plusieurs tensioactifs et/ou émulsifiants, tels que des tensioactifs non ioniques, cationiques, anioniques ou zwitterioniques, comme les copolymères séquencés amphiphiles, ou leurs mélanges.

15. Préparation pharmaceutique liquide selon la revendication 14, **caractérisée en ce qu'**un ou plusieurs des composés de la préparation pharmaceutique liquide est/sont solubilisé(s) dans un ou plusieurs tensioactifs choisis parmi
les agents tensioactifs non ioniques, comme les poloxamères, par exemple les copolymères séquencés de poly(oxypropylène)-poly(oxyéthylène), les polyoxyéthylène alkyl éthers, les dérivés d'huile de ricin contenant du polyoxyéthylène, les esters de sorbitane d'acide gras contenant du polyoxyéthylène, les mono- et di-glycérides et leurs esters, les stéarates de polyoxyéthylène, les polyglycérolesters d'acides gras (comme l'acide polyglycérolpolyricinoléique (PGPR)), et les esters de sorbitane d'acide gras,
les tensioactifs cationiques, comme les composés d'ammonium secondaire, quaternaire et tertiaire et les phospholipides cationiques,
les agents tensioactifs anioniques, comme les sels d'acide gras, les lactylates, en particulier le stéaroyllactylate de sodium et/ou de calcium, les sulfates d'alkyle, les sulfonates d'alkyle, le latanol, et les phospholipides anioniques, comme la phosphatidylsérine,
les agents tensioactifs zwitterioniques, comme les phospholipides zwitterioniques, tels que la phosphatidylcholine et la phosphatidyléthanolamine,
ou leurs mélanges,
de préférence des agents tensioactifs non ioniques ou leurs mélanges.

16. Préparation pharmaceutique liquide selon l'une quelconque des revendications 1 à 15, **caractérisée en ce qu'**elle comprend de la nicotine base, de l'hydrogéno-carbonate de sodium en tant qu'agent tampon, de l'eau en tant que solvant, un poloxamère en tant qu'agent tensioactif, de l'EDTA en tant qu'agent de chélation, de l'Acésulfame K en tant qu'édulcorant, facultativement un ou plusieurs conservateurs, et facultativement un ou plusieurs agents de saveur ou d'arôme.

17. Système permettant de délivrer de la nicotine à un sujet, comprenant une préparation pharmaceutique liquide selon l'une quelconque des revendications 1 à 16 et au moins un autre moyen permettant de délivrer de la nicotine à un sujet.

18. Système permettant d'obtenir la réduction de l'envie de fumer ou d'utiliser du tabac et/ou de fournir la sensation de la satisfaction de fumer sans fumer, comprenant une formulation pharmaceutique liquide selon l'une quelconque des revendications 1 à 16 et au moins un autre moyen permettant d'obtenir la réduction de l'envie de fumer ou d'utiliser du tabac.

19. Système selon l'une quelconque des revendications 17 et 18, dans lequel le au moins un autre moyen est choisi dans le groupe constitué par l'administration par l'intermédiaire de gommes à mâcher, de pulvérisations nasales, de timbres transdermiques, de dispositifs d'inhalation, de losanges, de comprimés et de moyens ou procédés parentéraux, de moyens ou procédés sous-cutanés, de moyens ou procédés intraveineux, de moyens ou procédés rectaux, de moyens ou procédés vaginaux et de moyens ou procédés à travers les muqueuses ; ou l'utilisation de tabac.

20. Système selon la revendication 19, dans lequel le au moins un autre moyen comprend l'administration de nicotine.

21. Procédé de fabrication d'une préparation pharmaceutique liquide selon l'une quelconque des revendications 1 à 16, comprenant les étapes suivantes :
a) la préparation d'un premier mélange comprenant un premier solvant, au moins un agent tampon et/ou d'autres moyens pour réguler le pH, et facultativement de la nicotine base,
b) facultativement l'ajout de premiers composants audit premier mélange, lesdits premiers composants ayant été facultativement solubilisés,
c) facultativement la préparation d'un ou de plusieurs second(s) mélange(s) comprenant un ou plusieurs second(s) solvant(s) et second(s) composant(s), pouvant comprendre de la nicotine base,
d) facultativement le mélange du premier mélange et du ou des second(s) mélange(s) facultatif(s) pour former un mélange final, facultativement en ajoutant un ou plusieurs solvant(s) supplémentaire(s), et facultativement en ajoutant de la nicotine base,
e) facultativement l'ajustement du pH du mélange final.

22. Procédé selon la revendication 21, dans lequel le mélange est réalisé à une température allant de la température ambiante à environ 95 °C.

23. Procédé selon l'une quelconque des revendications 21 et 22, dans lequel le tamponnage et/ou la régulation du pH est obtenu en utilisant un ou plusieurs agents tampons choisis dans le groupe constitué par un carbonate, tel qu'un monocarbonate, un bicarbonate ou un sesquicarbonate ; un glycinate, un phosphate, un glycérophosphate, un acétate, un gluconate ou un citrate de métal alcalin, tel que le potassium ou le sodium, ou d'ammonium, et leurs mélanges ; et/ou au moyen d'agents régulant le pH, tels que les agents choisis dans le groupe constitué par l'hydroxyde de sodium, l'hydroxyde de potassium, l'hydroxyde de calcium et l'oxyde de calcium ; et/ou en utilisant au moins partiellement des formes de nicotine régulant le pH.

24. Procédé selon l'une quelconque des revendications 21 à 23, dans lequel le premier solvant et le ou les second(s) solvant(s) facultatif(s) est/sont choisi(s) parmi l'eau et/ou un alcool, comme l'éthanol, le glycérol, le propylène glycol et le polyéthylène glycol, et/ou un ou plusieurs lipides ou leurs mélanges.

25. Procédé selon l'une quelconque des revendications 21 à 24, dans lequel les premiers et seconds composants facultatifs sont choisis parmi
un ou plusieurs agents de saveur et/ou aromatisants, tels que ceux choisis dans le groupe constitué par les huiles essentielles obtenues par distillations, extractions par solvant ou expressions à froid de fleurs, de bourgeons, de feuilles, de tiges, de fruits, de graines, de pelures, d'écorces ou de racines à l'état frais ou sec, par exemple l'huile de menthe poivrée, de menthe verte, d'eucalyptus, de gaulthérie, de niaouli, de clou de girofle, de cardamome, de cannelle, d'amande amère, de coriandre, de carvi, de gingembre, de genièvre, d'orange, d'orange amère, de citron, de pamplemousse, de mandarine, de bergamote, de thym, de fenouil et de romarin ;
les agents de saveurs et aromatiques naturels comprenant les solutions diluées d'huiles essentielles ou les concentrés de composants de saveur d'origine naturelle provenant, par exemple, de fruits, de baies, de noix, d'épices, de menthes, de tabac, de cacao, de café, de thé, de vanille, de réglisse, de caramel, de caramel anglais, de miel, de vin, de liqueurs et de décoctions ;
les agents de saveur et aromatiques synthétiques constitués de mélanges de produits chimiques comprenant des hydrocarbures, des alcools, des aldéhydes, des esters, des cétones, des éthers et des oxydes mélangés pour correspondre à la saveur naturelle, par exemple, de fruits, de baies, de noix, d'épices, de menthe, de tabac, de cacao, de café, de thé, de vanille, de réglisse, de caramel, de caramel anglais, de miel, de vin, de liqueurs ou de décoctions ;
et leurs mélanges ;
un ou plusieurs additifs stabilisants, tels que ceux choisis dans le groupe constitué par les antioxydants, comme la vitamine E, c'est-à-dire les tocophérols, la vitamine C, c'est-à-dire l'acide ascorbique et ses sels, le pyrosulfite de sodium, le butylhydroxytoluène, l'hydroxyanisole butylé ; et les conservateurs, comme les parabènes, le chlorure de benzalkonium, le chlorobutanol, l'alcool benzylique, l'alcool bêta-phényléthylique, le chlorure de cétylpyridinium ; et les agents de chélation, comme l'EDTA ; et les galates, comme le galate de propyle ;
un ou plusieurs additifs choisis dans le groupe constitué par
les agents épaississants, comme les polymères naturels, semi-synthétiques ou synthétiques, par exemple l'amidon et les dérivés d'amidon, la cellulose et les dérivés de cellulose, les polyéthylène glycols et leurs dérivés, les polyacrylates et les esters et éthers de polyvinyle ;
les exhausteurs, comme l'azone ;
les vitamines, comme les vitamines C et E ;
les minéraux, comme les fluorures, en particulier le fluorure de sodium, le monofluorophosphate de sodium et le fluorure stanneux ;
les anti-odeurs, comme le zinc et les cyclodextrines ;
les édulcorants, comme un ou plusieurs agents édulcorants synthétiques et/ou sucres naturels, tels que ceux choisis dans le groupe constitué par
les édulcorants artificiels, par exemple la saccharine et ses sels de sodium et de calcium, l'aspartame, l'acésulfame et son sel de potassium, la thaumatine et la glycyrrhizine ;
les alcools polyhydriques, tels que le sorbitol, le xylitol, le mannitol et le glycérol ; les monosaccharides, comme le glucose (également appelé dextrose), le fructose (également appelé lévulose) et le galactose ;
les disaccharides, comme le saccharose (également appelé sucrose), le lactose (également appelé sucre de lait) et le maltose (également appelé sucre de malt) ;
les mélanges de sucre, comme le sirop de glucose liquide, par exemple les hydrolysats d'amidon contenant un mélange composé principalement de dextrose, de maltose, de dextrines et d'eau, le sirop de sucre inverti, par exemple le saccharose inverti par une invertase contenant un mélange de dextrose, de lévulose et d'eau, les sirops à teneur élevée en sucre, tels que la mélasse, le miel et un extrait de malt ;
et leurs mélanges.

26. Procédé selon l'une quelconque des revendications 21 à 25, dans lequel le premier composant et/ou le second composant est/sont solubilisé(s) dans un ou plusieurs agents tensioactifs et/ou émulsifiants, tels que des tensioactifs non ioniques, cationiques, anioniques ou zwitterioniques, comme les copolymères séquencés amphiphiles, ou leurs mélanges, moyennant quoi, de préférence, le ou les agents tensioactifs est/sont choisi(s) parmi
des agents tensioactifs non ioniques, comme les poloxamères, par exemple les copolymères séquencés de poly(oxypropylène)-poly(oxyéthylène), les polyoxyéthylène alkyl éthers, les dérivés d'huile de ricin contenant du polyoxyéthylène, les esters de sorbitane d'acide gras contenant du polyoxyéthylène, les mono- et di-glycérides et leurs esters, les stéarates de polyoxyéthylène, les polyglycérolesters d'acides gras (comme l'acide polyglycérolpolyricinoléique (PGPR)), et les esters de sorbitane d'acide gras,
les tensioactifs cationiques, comme les composés d'ammonium secondaire, quaternaire et tertiaire et les phospholipides cationiques,
les agents tensioactifs anioniques, comme les sels d'acide gras, les lactylates, en particulier le stéaroyllactylate de sodium et/ou de calcium, les sulfates d'alkyle, les sulfonates d'alkyle, le latanol, et les phospholipides anioniques, comme la phosphatidylsérine,
les agents tensioactifs zwitterioniques, comme les phospholipides zwitterioniques, tels que la phosphatidylcholine et la phosphatidyléthanolamine,
ou leurs mélanges, moyennant quoi, de manière préférée entre toutes, les agents tensioactifs ou leurs mélanges sont non ioniques.

27. Préparation pharmaceutique liquide selon l'une quelconque des revendications 1 à 16, pour un usage en thérapie.

28. Préparation pharmaceutique liquide selon la revendication 27, dans laquelle la thérapie est le traitement d'une maladie choisie dans le groupe constitué par la dépendance au tabac ou à la nicotine, la maladie d'Alzheimer, la maladie de Crohn, la maladie de Parkinson, le syndrome de la Tourette, la rectocolite hémorragique ; et la surveillance du poids.

29. Utilisation de nicotine base pour la fabrication d'une préparation pharmaceutique liquide selon l'une quelconque des revendications 1 à 6, pour le traitement d'une maladie choisie dans le groupe constitué par la dépendance au tabac ou à la nicotine, la maladie d'Alzheimer, la maladie de Crohn, la maladie de Parkinson, le syndrome de la Tourette, la rectocolite hémorragique ; et la surveillance du poids.
